# EUROPEAN PATENT APPLICATION

(11) **EP 2 982 758 A1**
(43) Date of publication of application: **10.02.2016**
(21) Application number: 14179767.0
(22) Date of filing: 04.08.2014
(51) Int. Cl.: C12N 15/86, C12N 9/52, A61K 48/00

(54) **Genome editing for the treatment of huntington's disease**

(71) Applicant: Centre Hospitalier Universitaire Vaudois (CHUV), 1011 Lausanne (CH)
(72) Inventor: Déglon, Nicole, 1521 Curtilles (CH); Merienne, Nicolas, 1081 Montpreveyres (CH)
(74) Representative: KATZAROV S.A.

(57) **Abstract**

The invention relates to the treatment of Huntington's disease (HD) using the Clustered-Regularly Interspaced Short Palindromic Repeats (CRISPR) system. This technology offers the possibility to design a small RNA (sgRNA), which is incorporated into a CRISPR-associated protein (Cas9) to recognize and induce DNA double-strand breaks at a specific target location. In the context of HD, this allows to block the expression of the mutant huntingtin or repair the CAG expansion causing the disease.

SEQUENCE LISTING

<110>  Centre Hospitalier Universitaire Vaudois CHUV

<120>  GENOME EDITING FOR THE TREATMENT OF HUNTINGTON's DISEASE

<130>  19685/EP

<160>  14

<170>  PatentIn version 3.5

<210>  1
<211>  82
<212>  DNA
<213>  Artificial Sequence

<220>
<223>  synthetic sequence

<400>  1
gttttagagc tagaaatagc aagttaaaat aaggctagtc cgttatcaac ttgaaaaagt      60

ggcaccgagt cggtgctttt tt                                                82


<210>  2
<211>  20
<212>  DNA
<213>  Artificial Sequence

<220>
<223>  synthetic sequence

<400>  2
gaccctggaa aagctgatga                                                   20


<210>  3
<211>  20
<212>  DNA
<213>  Artificial Sequence

<220>
<223>  synthetic sequence

<400>  3
gaagttcgag ggcgacaccc                                                   20


<210>  4
<211>  20
<212>  DNA
<213>  Artificial Sequence

<220>
<223>  synthetic sequence

<400>  4
gaccctggaa aagctgatga                                                   20


<210>  5

<211>  20
<212>  DNA
<213>  Artificial Sequence

<220>
<223>  synthetic sequence

<400>  5
gtggatgaca taatgctttt                                                   20


<210>  6
<211>  20
<212>  DNA
<213>  Artificial Sequence

<220>
<223>  synthetic sequence


<220>
<221>  misc_feature
<222>  (4)..(4)
<223>  n=G or A

<400>  6
gganccgctg caccgaccgt                                                   20


<210>  7
<211>  20
<212>  DNA
<213>  Artificial Sequence

<220>
<223>  synthetic sequence

<400>  7
tgggacgcaa ggcgccgtgg                                                   20


<210>  8
<211>  4140
<212>  DNA
<213>  Artificial Sequence

<220>
<223>  synthetic sequence

<400>  8
   atggacaaga agtactccat tgggctcgat atcggcacaa acagcgtcgg ctgggccgtc      60
   attacggacg agtacaaggt gccgagcaaa aaattcaaag ttctgggcaa taccgatcgc     120
   cacagcataa agaagaacct cattggcgcc ctcctgttcg actccgggga gacggccgaa     180
   gccacgcggc tcaaaagaac agcacggcgc agatataccc gcagaaagaa tcggatctgc     240
   tacctgcagg agatctttag taatgagatg gctaaggtgg atgactcttt cttccatagg     300
   ctggaggagt cctttttggt ggaggaggat aaaaagcacg agcgccaccc aatctttggc     360
   aatatcgtgg acgaggtggc gtaccatgaa aagtacccaa ccatatatca tctgaggaag     420
   aagcttgtag acagtactga taaggctgac ttgcggttga tctatctcgc gctggcgcat 480
   atgatcaaat ttcggggaca cttcctcatc gagggggacc tgaacccaga caacagcgat 540
   gtcgacaaac tctttatcca actggttcag acttacaatc agcttttcga agagaacccg 600
   atcaacgcat ccggagttga cgccaaagca atcctgagcg ctaggctgtc caaatcccgg 660
   cggctcgaaa acctcatcgc acagctccct ggggagaaga agaacggcct gtttggtaat 720
   cttatcgccc tgtcactcgg gctgaccccc aactttaaat ctaacttcga cctggccgaa 780
   gatgccaagc ttcaactgag caaagacacc tacgatgatg atctcgacaa tctgctggcc 840
   cagatcggcg accagtacgc agaccttttt ttggcggcaa agaacctgtc agacgccatt 900
   ctgctgagtg atattctgcg agtgaacacg gagatcacca aagctccgct gagcgctagt 960
   atgatcaagc gctatgatga gcaccaccaa gacttgactt tgctgaaggc ccttgtcaga 1020
   cagcaactgc ctgagaagta caaggaaatt ttcttcgatc agtctaaaaa tggctacgcc 1080
   ggatacattg acggcggagc aagccaggag gaattttaca aatttattaa gcccatcttg 1140
   gaaaaaatgg acggcaccga ggagctgctg gtaaagctta acagagaaga tctgttgcgc 1200
   aaacagcgca ctttcgacaa tggaagcatc ccccaccaga ttcacctggg cgaactgcac 1260
   gctatcctca ggcggcaaga ggatttctac ccctttttga aagataacag ggaaaagatt 1320
   gagaaaatcc tcacatttcg gataccctac tatgtaggcc ccctcgcccg gggaaattcc 1380
   agattcgcgt ggatgactcg caaatcagaa gagaccatca ctccctggaa cttcgaggaa 1440
   gtcgtggata agggggcctc tgcccagtcc ttcatcgaaa ggatgactaa ctttgataaa 1500
   aatctgccta acgaaaaggt gcttcctaaa cactctctgc tgtacgagta cttcacagtt 1560
   tataacgagc tcaccaaggt caaatacgtc acagaaggga tgagaaagcc agcattcctg 1620
   tctggagagc agaagaaagc tatcgtggac ctcctcttca agacgaaccg gaaagttacc 1680
   gtgaaacagc tcaaagaaga ctatttcaaa aagattgaat gtttcgactc tgttgaaatc 1740
   agcggagtgg aggatcgctt caacgcatcc ctgggaacgt atcacgatct cctgaaaatc 1800
   attaaagaca aggacttcct ggacaatgag gagaacgagg acattcttga ggacattgtc 1860
   ctcaccctta cgttgtttga agatagggag atgattgaag aacgcttgaa aacttacgct 1920
   catctcttcg acgacaaagt catgaaacag ctcaagaggc gccgatatac aggatggggg 1980
   cggctgtcaa gaaaactgat caatgggatc cgagacaagc agagtggaaa gacaatcctg 2040
   gatttcttta agtccgatgg atttgccaac cggaacttca tgcagttgat ccatgatgac 2100
   tctctcacct ttaaggagga catccagaaa gcacaagttt ctggccaggg ggacagtctt 2160
   cacgagcaca tcgctaatct tgcaggtagc ccagctatca aaaagggaat actgcagacc 2220
   gttaaggtcg tggatgaact cgtcaaagta atggaaggca ataagcccga gaatatcgtt 2280
   atcgagatgg cccgagagaa ccaaactacc cagaagggac agaagaacag tagggaaagg 2340
   atgaagagga ttgaagaggg tataaaagaa ctggggtccc aaatccttaa ggaacaccca 2400
   gttgaaaaca cccagcttca gaatgagaag ctctacctgt actacctgca gaacggcagg 2460
   gacatgtacg tggatcagga actggacatc aatcggctct ccgactacga cgtggatcat 2520
   atcgtgcccc agtcttttct caaagatgat tctattgata ataaagtgtt gacaagatcc 2580
   gataaaaata gagggaagag tgataacgtc ccctcagaag aagttgtcaa gaaaatgaaa 2640
   aattattggc ggcagctgct gaacgccaaa ctgatcacac aacggaagtt cgataatctg 2700
   actaaggctg aacgaggtgg cctgtctgag ttggataaag ccggcttcat caaaaggcag 2760
   cttgttgaga cacgccagat caccaagcac gtggcccaaa ttctcgattc acgcatgaac 2820
   accaagtacg atgaaaatga caaactgatt cgagaggtga aagttattac tctgaagtct 2880
   aagctggtct cagatttcag aaaggacttt cagttttata aggtgagaga gatcaacaat 2940
   taccaccatg cgcatgatgc ctacctgaat gcagtggtag gcactgcact tatcaaaaaa 3000
   tatcccaagc ttgaatctga atttgtttac ggagactata aagtgtacga tgttaggaaa 3060
   atgatcgcaa agtctgagca ggaaataggc aaggccaccg ctaagtactt cttttacagc 3120
   aatattatga attttttcaa gaccgagatt acactggcca atggagagat tcggaagcga 3180
   ccacttatcg aaacaaacgg agaaacagga gaaatcgtgt gggacaaggg tagggatttc 3240
   gcgacagtcc ggaaggtcct gtccatgccg caggtgaaca tcgttaaaaa gaccgaagta 3300
   cagaccggag gcttctccaa ggaaagtatc ctcccgaaaa ggaacagcga caagctgatc 3360
   gcacgcaaaa aagattggga ccccaagaaa tacggcggat tcgattctcc tacagtcgct 3420
   tacagtgtac tggttgtggc caaagtggag aaagggaagt ctaaaaaact caaaagcgtc 3480
   aaggaactgc tgggcatcac aatcatggag cgatcaagct tcgaaaaaaa ccccatcgac 3540
   tttctcgagg cgaaaggata taaagaggtc aaaaaagacc tcatcattaa gcttcccaag 3600
   tactctctct ttgagcttga aaacggccgg aaacgaatgc tcgctagtgc gggcgagctg 3660
   cagaaaggta acgagctggc actgccctct aaatacgtta atttcttgta tctggccagc 3720
   cactatgaaa agctcaaagg gtctcccgaa gataatgagc agaagcagct gttcgtggaa 3780
   caacacaaac actaccttga tgagatcatc gagcaaataa gcgaattctc caaaagagtg 3840
   atcctcgccg acgctaacct cgataaggtg ctttctgctt acaataagca cagggataag 3900
   cccatcaggg agcaggcaga aaacattatc cacttgttta ctctgaccaa cttgggcgcg 3960
   cctgcagcct tcaagtactt cgacaccacc atagacagaa agcggtacac ctctacaaag 4020
   gaggtcctgg acgccacact gattcatcag tcaattacgg ggctctatga aacaagaatc 4080
   gacctctctc agctcggtgg agacagcagg gctgacccca agaagaagag gaaggtgtga 4140

<210>  9
<211>  972
<212>  DNA
<213>  Artificial Sequence

<220>
<223>  synthetic sequence

<400>  9
ctgcccggcg cctacaacgt caacatcaag ttggacatca cctcccacaa cgaggactac      60

accatcgtgg aacagtacga acgcgccgag ggccgccact ccaccggcgg catggacgag     120

ctgtacaagg gtaccagcgc gccgcgtcga gagatggggg aggctaacag aagtgtacgc     180

gttggctgct gtcaggggta ccatggacgc gtagacaagg tgaggaacct agctcagtca     240

tgcaccgggc tgcagatgag caagggcgag gagctgttca ccggggtggt gcccatcctg     300

gtcgagctgg acggcgacgt aaacggccac aagttcagcg tgtccggcga gggcgagggc     360

gatgccacct acggcaagct gaccctgaag ttcatctgca ccaccggcaa gctgcccgtg     420

ccctggccca ccctcgtgac caccctgacc tacggcgtgc agtgcttcag ccgctacccc     480

gaccacatga agcagcacga cttcttcaag tccgccatgc ccgaaggcta cgtccaggag     540

cgcaccatct tcttcaagga cgacggcaac tacaagaccc gcgccgaggt gaagttcgag     600

ggcgacaccc tggtgaaccg catcgagctg aagggcatcg acttcaagga ggacggcaac     660

atcctggggc acaagctgga gtacaactac aacagccaca acgtctatat catggccgac     720

aagcagaaga acggcatcaa ggccaacttc aagatccgcc acaacatcga ggacggcagc     780

gtgcagctcg ccgaccacta ccagcagaac acccccatcg gcgacggccc cgtgctgctg     840

cccgacaacc actacctgag cacccagtcc gccctgagca aagaccccaa cgagaagcgc     900

gatcacatgg tcctgctgga gttcgtgacc gccgccggga tcactcacgg catggacgag     960

ctgtacaagt ga                                                         972


<210>  10
<211>  51
<212>  DNA
<213>  Homo sapiens


<220>
<221>  misc_feature
<222>  (26)..(26)
<223>  n=A or G

<400>  10
agcgtctggg acgcaaggcg ccgtgngggc tgccgggacg ggtccaagat g               51


<210>  11
<211>  51
<212>  DNA
<213>  Homo sapiens

<220>
<221>  misc_feature
<222>  (26)..(26)
<223>  n=A or C

<400>  11
cagcagcagc agcagcagca gcagcnacag ccgccaccgc cgccgccgcc g                51


<210>  12
<211>  51
<212>  DNA
<213>  Homo sapiens


<220>
<221>  misc_feature
<222>  (26)..(26)
<223>  n=A or G

<400>  12
agcagcagca gcagcaacag ccgccnccgc cgccgccgcc gccgccgcct c                51


<210>  13
<211>  51
<212>  DNA
<213>  Homo sapiens


<220>
<221>  misc_feature
<222>  (26)..(26)
<223>  n=A or C

<400>  13
cgacacttca cacacagctt cgcctnaccc cattacagtc tcaccacgcc c                51


<210>  14
<211>  4533
<212>  DNA
<213>  Homo sapiens

<400>  14
cttactccct gctgggcagg ctggacagct gccctctccg tttgcccctc taccacccaa        60

aaggcaggag gctctggaga ccaggaccct gcccgccacg gcctgtgtcc caggcgtgag       120

ggggtgcccc acagacctct gctgagctgc tgctgaatga cgccccttgg gggtcctgcc       180

ggaaggtcag agcaggggtg cactcccata aagaaacgcc cccaggtcgg gactcattcc       240

tgtgggcggc atcttgtggc catagctgct tctcgctgca ctaatcacag tgcctctgtg       300

ggcagcaggc gctgaccacc caggcctgcc ccagaccctc tcctcccttc cggggcgctg       360

cgctgggacc gatggggggc gccaggcctg tggacaccgc cctgcagggg cctctccagc       420

tcactggggg tggggtgggg gtcacacttg gggtcctcag gtcgtgccga ccacgcgcat       480

tctctgcgct ctgcgcagga gctcgcccac cctctccccg tgcagagagc cccgcagctg 540
gctccccgca gggctgtccg ggtgagtatg gctctggcca cgggccagtg tggcgggagg 600
gcaaacccca aggccacctc ggctcagagt ccacggccgg ctgtcgcccc gctccaggcg 660
tcggcggggg atcctttccg catgggcctg cgcccgcgct cggcgccccc tccacggccc 720
cgccccgtcc atggccccgt ccttcatggg cgagcccctc catggccctg cccctccgcg 780
ccccacccct ccctcgcccc acctctcacc ttcctgcccc gcccccagcc tccccacccc 840
tcaccggcca gtcccctccc ctatcccgct ccgcccctca gccgccccgc ccctcagccg 900
gcctgcctaa tgtccccgtc cccagcatcg ccccgccccg cccccgtctc gccccgcccc 960
tcaggcggcc tccctgctgt gccccgcccc ggcctcgcca cgcccctacc tcaccacgcc 1020
ccccgcatcg ccacgccccc cgcatcgcca cgcctccctt accatgcagt cccgccccgt 1080
cccttcctcg tcccgcctcg ccgcgacact tcacacacag cttcgcctca ccccattaca 1140
gtctcaccac gccccgtccc ctctccgttg agccccgcgc cttcgcccgg gtggggcgct 1200
gcgctgtcag cggcttgctg tgtgaggcag aacctgcggg ggcaggggcg ggctggttcc 1260
ctggccagcc attggcagag tccgcaggct agggctgtca atcatgctgg ccggcgtggc 1320
cccgcctccg ccggcgcggc cccgcctccg ccggcgcagc gtctgggacg caaggcgccg 1380
tgggggctgc cgggacgggt ccaagatgga cggccgctca ggttctgctt ttacctgcgg 1440
cccagagccc cattcattgc cccggtgctg agcggcgccg cgagtcggcc cgaggcctcc 1500
ggggactgcc gtgccgggcg ggagaccgcc atggcgaccc tggaaaagct gatgaaggcc 1560
ttcgagtccc tcaagtcctt ccagcagcag cagcagcagc agcagcagca gcagcagcag 1620
cagcagcagc agcagcagca acagccgcca ccgccgccgc cgccgccgcc gcctcctcag 1680
cttcctcagc cgccgccgca ggcacagccg ctgctgcctc agccgcagcc gcccccgccg 1740
ccgcccccgc cgccacccgg cccggctgtg gctgaggagc cgctgcaccg accgtgagtt 1800
tgggcccgct gcagctccct gtcccggcgg gtcccaggct acggcgggga tggcggtaac 1860
cctgcagcct gcgggccggc gacacgaacc cccggccccg cagagacaga gtgacccagc 1920
aacccagagc ccatgaggga cacccgcccc ctcctggggc gaggccttcc cccacttcag 1980
ccccgctccc tcacttgggt cttcccttgt cctctcgcga ggggaggcag agccttgttg 2040
gggcctgtcc tgaattcacc gaggggagtc acggcctcag ccctctcgcc cttcgcagga 2100
tgcgaagagt tggggcgaga acttgtttct ttttatttgc gagaaaccag ggcgggggtt 2160
cttttaactg cgttgtgaag agaacttgga ggagccgaga tttgctcagt gccacttccc 2220
tcttctagtc tgagagggaa gagggctggg ggcgcgggac acttcgagag gaggcggggt 2280
ttggagctgg agagatgtgg gggcagtgga tgacataatg cttttaggac gcctcggcgg 2340
gagtggcggg gcaggggggg ggcggggagt gagggcgcgt ccaatgggag atttcttttc 2400

ctagtggcac ttaaaacagc ctgagatttg aggctcttcc tacattgtca ggacatttca 2460

tttagttcat gatcacggtg gtagtaacac gattttaagc accacctaag agatctgctc 2520

atctaagcct aagttggtct gcaggcgttt gaatgagttg tggttgccaa gtaaagtggt 2580

gaacttacgt ggtgattaat gaaattatct taaatattag gaagagttga ttgaagtttt 2640

ttgcctatgt gtgttgggaa taaaaccaac acgttgctga tggggaggtt aattgccgag 2700

ggatgaatga ggtgtacatt ttaccagtat tccagtcagg cttgccagaa tacggggggt 2760

ccgcagactc cgtgggcatc tcagatgtgc cagtgaaagg gtttctgttt gcttcattgc 2820

tgacagcttg ttactttttg gaagctaggg gtttctgttg cttgttcttg gggagaattt 2880

ttgaaacagg aaaagagaga ccattaaaac atctagcgga accccaggac tttccctgga 2940

agtctgtgtg tcgagtgtac agtaggagtt aggaagtact ctggtgcagt tcaggccttt 3000

ctcttacctc tcagtattct atttccgatc tggatgtgtc ccagatggca tttggtaaga 3060

atatctctgt taagactgat taatttttag taatatttct tgttctttgt ttctgttatg 3120

atccttgtct cgtcttcaaa gtttaattag aaaatgattc ggagagcagt gttagcttat 3180

ttgttggaat aaaatttagg aataaattat tctaaaggat ggaaaaactt tttggatatt 3240

tggagaaatt ttaaaacaat ttggcttatc tcttcagtaa gtaatttctc atccagaaat 3300

ttactgtagt gcttttctag gaggtaggtg tcataaaagt tcacacattg catgtatctt 3360

gtgtaaacac taaacagggc tcctgatggg aaggaagacc tttctgctgg gctgcttcag 3420

acacttgatc attctaaaaa tatgccttct ctttcttatg ctgatttgac agaacctgca 3480

tttgcttatc ttcaaaatat gggtatcaag aaatttcctt tgctgccttg acaaaggaga 3540

tagattttgt ttcattactt taaggtaata tatgattacc ttatttaaaa aatttaatca 3600

ggactggcaa ggtggcttac acctttaatc cgagcacttt gggaggccta ggtggacgaa 3660

tcacctgagg tcaggagttt gagaccagcc tggctaacat ggtgaaaccc tgtctctact 3720

aaaaatacaa aaattagctg gtcatggtgg cacgtgcctg taatccaagc tacctgggag 3780

gctgaggcag gaaaatcgct tgaacccggg aggcagagtc tgcagtgagt tgagatcacg 3840

ccactgcact ccagcctggg tgacagagcg agactctatc tcaaaaaaaa ttttttttaa 3900

tgtattattt ttgcataagt aatacattga catgatacaa attctgtaat tacaaaaggg 3960

caataattaa aatatcttcc ttccacccct ttcctctgag tacctaactt tgtccccaag 4020

aacaagcact atttcagttc ctcatgtatc ctgccagata taacctgttc atattgtaag 4080

atagatttaa aatgctctaa aaacaaaagt agtttagaat aatatatatc tatatatttt 4140

ttgagatgta gtctcacatt gtcacccagg ctggagtgca gtgatacaat ctcggctcac 4200

tgcagtctct gcctcccagg ttcaaatgct tctcctgcct cagccttctg agtagctggg 4260

attacaggcg cccaccacca tgtccagcta atttttgtat ttttagtaga gatggggttt 4320

caccatgttg gccaggctgg tcttgaactc ctgaccttgt gatctgtcca cctcggcctc 4380

ccaaagtgct gggattacag gtgtgagcca ccatgcctgg ctagaataat aacttttaaa 4440

ggttcttagc atgctctgaa atcaactgca ttaggtttat ttatagtttt atagttattt 4500

taaataaaat gcatatttgt catatttctc tgt 4533

## Description

### FIELD OF THE INVENTION

The invention relates to the treatment of Huntington's disease (HD) using the Clustered-Regularly Interspaced Short Palindromic Repeats (CRISPR) system. This technology offers the possibility to design a small RNA (sgRNA), which is incorporated into a CRISPR-associated protein (Cas9) to recognize and induce DNA double-strand breaks at a specific target location. DNA double-strand breaks will be repaired by cellular machinery either by non-homologous end joining (NHEJ) or homologous recombination (HR) in the presence of a donor sequence for HD gene repair. In the context of HD, this allows to block the expression of the mutant huntingtin or repair the CAG expansion causing the disease.

### BACKGROUND OF THE INVENTION

Huntington's disease (HD) is a monogenic neurodegenerative disease characterized by a global impairment leading to death in 15-20 years. Although precise mechanisms leading to neuronal death are not yet understood, a pathologic expansion of CAG repeats (more than 40 CAG) at the end of the exon 1 of the huntingtin gene (*HTT*) has been identified as the cause of HD. In HD, the huntingtin protein (HTT) forms aggregates in cells, which interfere with normal cellular functions (1). In addition, normal function of HTT is altered and new pathologic interactions of mutant HTT (mHTT) with partners also participate to the neuronal death observed in HD (2). Currently, there are no curative treatments for HD but experimental approaches based on drug, cell and gene therapy are under investigation (3). One of the most promising is the silencing of *mHTT* with small-hairpin RNA (shRNA), which will degrade *mHTT* mRNA (4, 5).

Recently, approaches modulating *HTT* gene expression have also been described, in particular with zinc finger repressors (ZFP) and TALE-repressors (TALE-FP). Allele and non-allele specific targeting of mutant *HTT* using ZFP and TALE-repressors were used to block the expression of the mutant *HTT* (6). ZFP targeting the expanded CAG were shown to preferentially repress the expression of the human *HTT* containing longer CAG repeats *in vitro.* In the R6/2 mice, the injection of an AAV2/1 vector expressing ZF11xHunt-Kox-1 reduces the level of mutant *HTT* mRNA and the accumulation of misfolded HTT in the striatum by 40% on average. Clasping behavior and motor coordination were also improved (6).

Based on the same principle, WO 2013/130824 (Sangamo Biosciences, Inc.) discloses methods and compositions for treating or preventing Huntington's disease. In particular, this document provides methods and compositions for modulating expression of a mutant *HTT* allele so as to treat Huntington's disease. Also provided are methods and compositions for generating animal models of Huntington's disease. The invention is based on ZFN and TALE with engineered DNA binding domain targeting the *HTT* gene. These proteins are in operative linkage with regulatory (or functional domain) as part of a fusion protein. The functional domain can be a transcriptional activation domain or a transcriptional repression domain. These strategies will therefore either repress or activate the expression of the human *HTT* gene (global or allele-specific).

The recently described method based on the bacterial clustered regularly interspaced short palindromic repeats (CRISPR) system is a promising tool for gene editing in mammals and offers a unique opportunity to irreversibly modify the *HTT* gene itself and develop therapeutic strategies based on DNA editing/repair (7). The CRISPR system contains two components: (i) an artificial single guide RNA (sgRNA) with approximately 20 nucleotides recognizing the target sequence stabilized by a loop (tracrRNA) and (ii) the CRISPR-associated protein Cas9 nuclease cleaving at the target site. Any sequence of approximately 23 nucleotides including the protospacer adjacent motif (PAM = NGG or NAG for Cas9)) could be a CRISPR target sequence, which provides a large number of potential targets for each gene and greatly facilitates the development of DNA repair strategies compared to the other systems. Sternberg and collaborators demonstrated that both binding and cleavage of DNA by Cas9 require recognition of the short trinucleotide PAM (8). The DNA endonuclease binds targeted nucleotides to perform double-strand breaks (DSB) and non-homologous end joining (NHEJ) with insertions or deletions (indels) in the sequence created by the cellular repair machinery. When an exogenous DNA sequence with homology with the target sequence is added, HR (HR) and integration into the desired locus is occurring after the DSB.

One prerequisite for the development of *in vivo* gene repair strategy in the CNS is an efficient delivery system. The design, production, and efficiency of gene transfer vectors, has improved remarkably over time, leading to safer transduction and long-term and robust transgene expression in the brain (9). This has led to the initiation of several phase I/II clinical trials with adeno-associated vectors (AAV) and lentiviral vectors (LV) in patients suffering from Parkinson's, Alzheimer's, Batten, adrenoleukodystrophy or Canavan's disease (10-12). The identification of numerous natural or chimeric AAV serotypes with variable capsid proteins has been exploited to modulate the entry into the host and enhance transduction efficiencies in the CNS (13, 14). Most studies with LV have been performed with VSV-G (vesicular stomatitis virus glycoprotein G) pseudotyped vectors, but the tropism of the vector could be altered with the use of heterologous envelopes. Their relatively large cloning capacity (8-9 kb compared with 4-5kb for AAV) is particularly advantageous for the cloning of complex expression cassettes with large cDNAs.

Current approaches based on HTT silencing (RNAi, ASO) or modulation of HTT expression (ZFN/TALEN) required a continuous expression of the compounds to maintain the therapeutic benefits. In contrast, the CRISPR/Cas9 system is inducing indels or DNA repair. These reactions are permanent and irreversible and will ensure a long-lasting therapeutic benefit.

### BRIEF DESCRIPTION OF THE INVENTION

One of the objects of the present invention is to provide a kit for the treatment of Huntington's disease (HD) for allele or non-allele-specific huntingtin (*HTT*) gene editing or repair comprising:
a gene delivery vector consisting of at least one viral vector selected among adeno-associated vectors (AAV) and/or lentiviral vectors (LV);
a Cas9 being human codon-optimized ;
an artificial single guide RNA (sgRNA) having a total size from 63-115 nucleotides comprising a crRNA sequence of 15-30 nucleotides recognizing the *HTT* gene and capable of targeting around the ATG +/- 4 kb of the *HTT* gene wherein said sgRNA further comprises a tracrRNA sequence of 48-85 nucleotides long.

Another object of the present invention is to provide a kit for the treatment of Huntington's disease, for use in a method for non-allele-specific *HTT* inactivation of the human *HTT* wild-type (WT) and mutant alleles.

A further object of the invention is to provide a kit for the treatment of Huntington's disease, for use in a method for non-allele-specific *HTT* gene repair based on HR with a DNA template containing a WT *HTT* sequence.

Also provided is a kit for the treatment of Huntington's disease, for use in a method for allele-specific *HTT* inactivation of the human mutant *HTT* gene characterized in that the sgRNA of said kit is capable of recognizing SNP sequences located around the ATG of the *HTT* gene of +/- 4 kb and with a high frequency of heterozygosity in the human population.

The present invention also provide a kit for the treatment of Huntington's disease, for use in a method for mutant *HTT* gene repair based on HR with a DNA template containing a WT *HTT* sequence characterized in that the sgRNA of said kit is capable of recognizing SNP sequences located around the ATG of the *HTT* gene of +/- 4 kb and with a high frequency of heterozygosity in the human population.

Other objects and advantages of the invention will become apparent to those skilled in the art from a review of the ensuing detailed description, which proceeds with reference to the following illustrative drawings, and the attendant claims.

Applicants use the CRISPR system to definitely repair the HD mutation. Applicants target sites around the transcription start site and the translation initiation codon ATG with the CRISPR to block the expression of the mutant *HTT* at the DNA level. Both Cas9 and sgRNAs are delivered in striatal neurons of mice expressing the human mutant *HTT* with virals vectors, for example VSV-G pseudotyped LV or AAV2/5 vector, expressing a human codon optimized Cas9 under the PGK or CMV or CBA promoter followed by the woodchuck post-transcriptional regulatory element (WPRE). The second one expresses one copy or multiple sgRNA under the H1 promoter or other polymerase II or III promoters and stuffer DNA to ensure independent expression of each sgRNA. Blocking the expression of *mHTT* in adult neurons helps to rescue the HD phenotype and therefore provides therapeutic benefit.

DNA disruption/repair of Huntington's disease mutation using the CRISPR/Cas9 system represents a new and original therapeutic approach. The present invention offers the possibility to act at the DNA level with engineered nucleases to inactivate or repair a disease-causing mutation. Previous works using RNA silencing obtained a significant decrease of *mHTT* expression, which lead to an improved phenotype. Recently, an approach based on zinc-finger repressors was evaluated in HD mouse model brain and reached a low but significant reduction of *mHTT* expression. However, all these approaches need a continuous expression of the therapeutic gene. In the case of the DNA repair strategy of the present invention, only a transient expression of the nuclease sufficient to induce the DSB is required. Repair mediated by this DSB will be permanent.

### BRIEF DESCRIPTION OF THE FIGURES

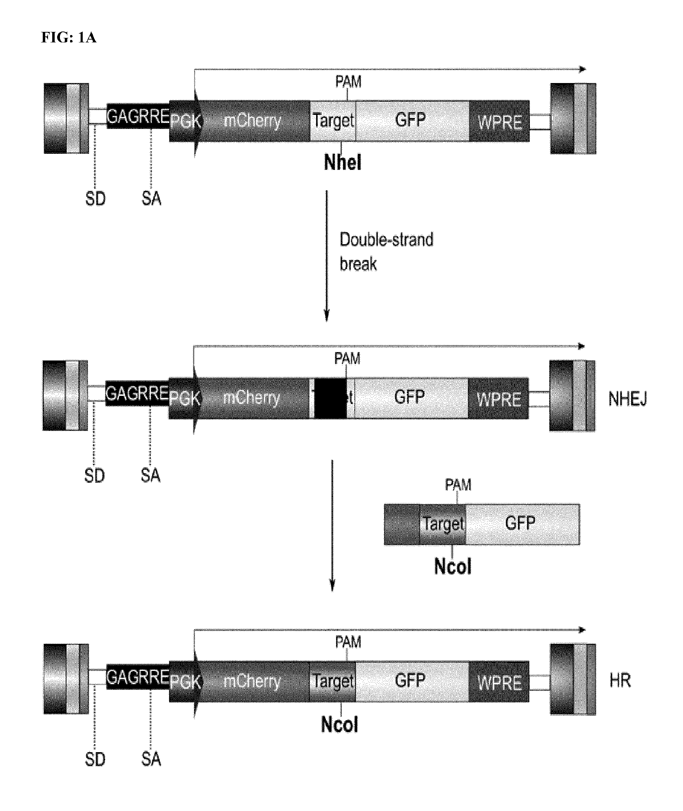

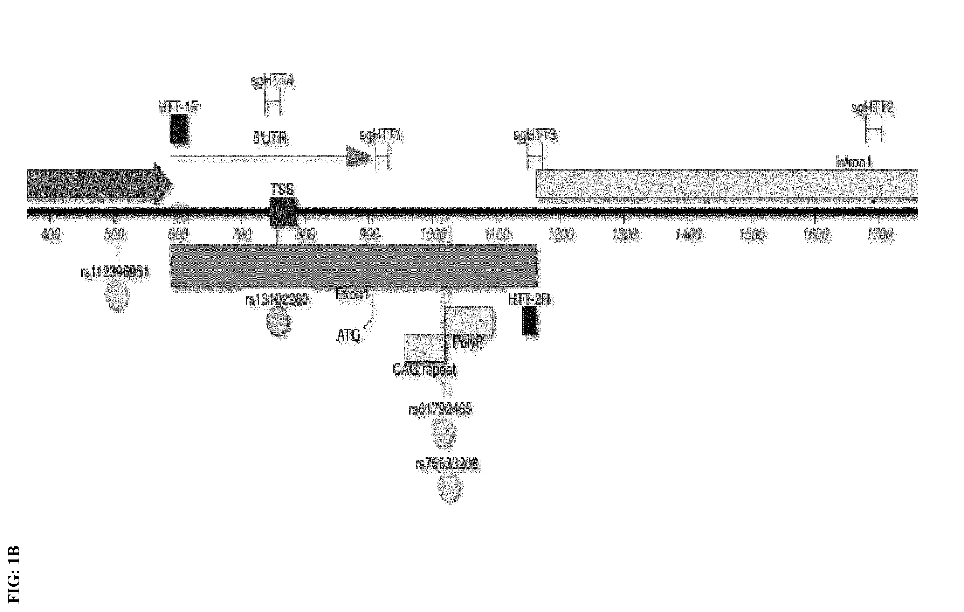

Figure 1: Description of the constructs used for the experiments. (**A**) An artificial target sequence was used and cloned in viral vectors. An artificial target sequence was introduced between the mCherry-GFP sequence expressed under the PGK promoter. This target consists of 20 nucleotides carrying a STOP codon two nucleotides 5' of the NGG PAM. Cells expressing this construct will display only mCherry fluorescence. If DSB occurs after target sequence recognition by the CRISPR system, the STOP codon will be removed and a fusion protein: mCherry-GFP will be expressed. For HR experiments, the presence of the homologous donor DNA without the STOP codon will be integrated at the desire locus. In addition, HR leads to the insertion of an NcoI restriction site at the end of the target sequence. (**B**) Schematic representation of the human *HTT* gene.

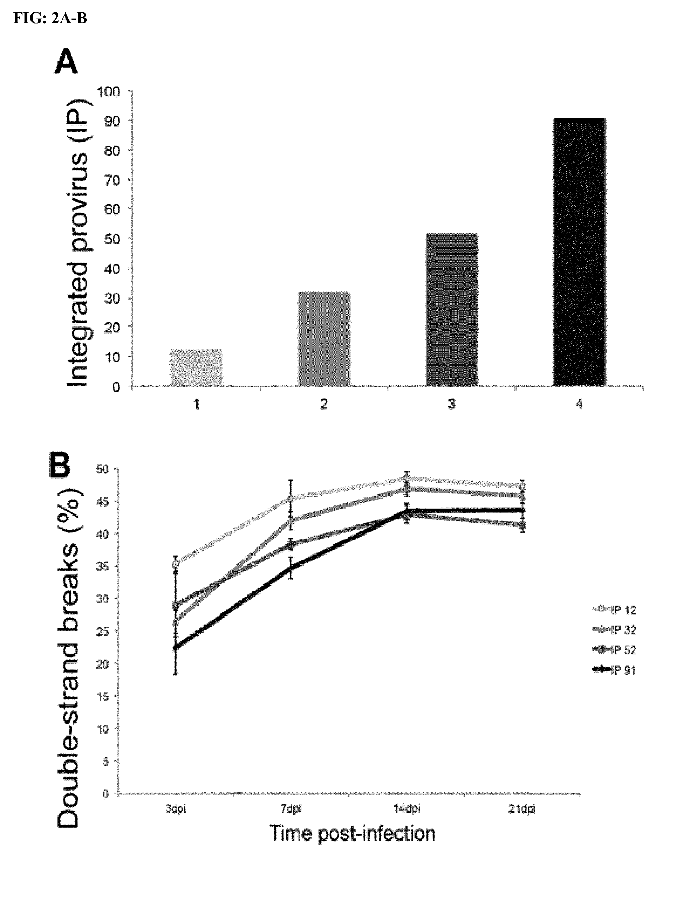

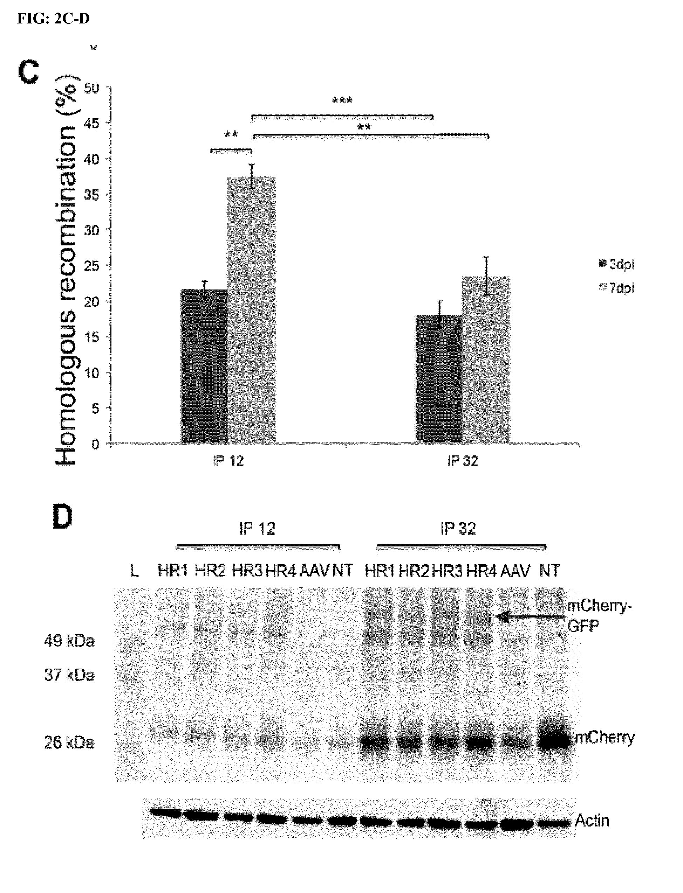

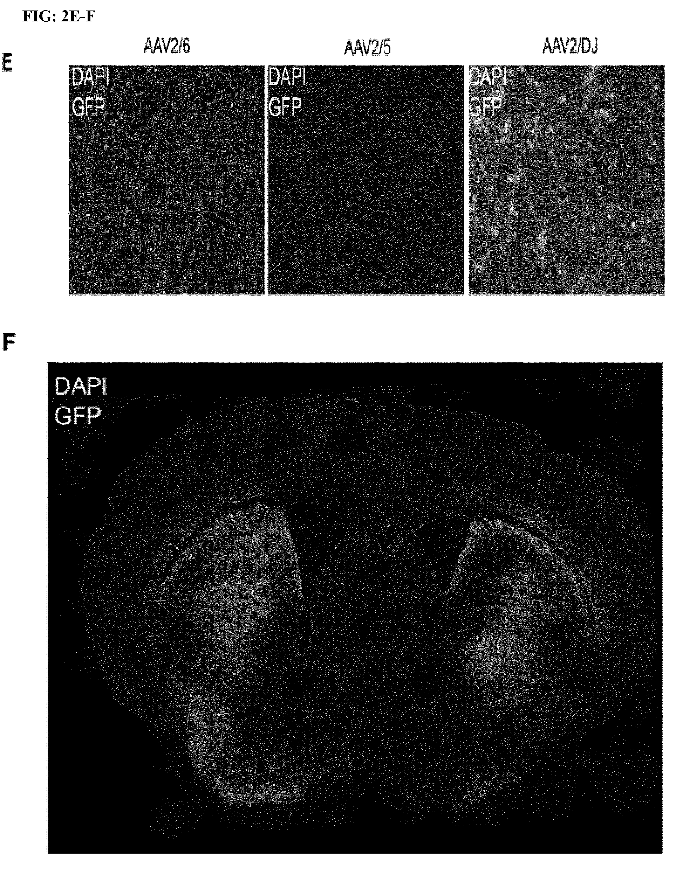

Figure 2: Optimization of Cas9 and sgRNA delivery *in vitro.* (**A**) HEK-293T cells were infected with increasing amounts of LV-TARGET (MOI 1, 10, 50 and 100) to produce HEK-TARGET cells. FACS analysis of mCherry fluorescence was used to select the corresponding cell populations. The number of integrated provirus was determined by qPCR on genomic DNA. The four cell populations contain on average 12, 32, 52 and 91 integrated target sequences, respectively. (**B**) Equal amounts of LV-hCas9 and LV-sgTARGET were incubated on 300'000 cells of the 4 populations. Genomic DNA was extracted 3, 7, 14 and 21 days post-infection to perform surveyor mutation detection assay. Results show that the amount of DSB increases with time in all the populations (Time effect, factorial ANOVA, p < 0.01). Cells containing low numbers of target sequences reach a maximal DSB earlier than cells containing higher copies of target sequence, (MOI effect, factorial ANOVA, p < 0.01). Results are presented as mean ± SEM. (**C**) HR was evaluated in HEK-TARGET cells (MOI 12 and 32) by transient transfection of plasmids encoding hCas9, the sgTARGET and the homologous donor DNA. Genomic DNA was extracted 3 and 7 days post-transfection to perform surveyor mutation detection assay and NcoI RFLP to evaluate the percent of HR. Surveyor analysis reveal up to 45% of DSB at 7 days post-transfection (data not shown). NcoI digestion displays an efficient HR at the expected locus 3 days post-transfection. Efficiency of HR increased with time to reach up to 37.5% at 7 days post-transfection. Results are presented as mean ± SEM. ** p < 0.01, *** p < 0.001. (**D**) Results obtained for HR experiments were confirmed by western blots using anti-mCherry antibody. The presence of a band around 54 kDa corresponding to the molecular weight of mCherry-GFP fusion protein is observed in treated samples. Actin was used as a control for normalization. HR1-4: four replicates transfected with plasmids expressing hCas9, sgTARGET and the donor DNA. AAV: cells transfected with the donor DNA and an empty plasmid. NT: untransfected cells. (**E**) E17 rat primary neuronal cells were used to compare the transduction efficiency of AAV2/5, AAV2/6 and AAV2/DJ expressing the GFP reporter gene under the chicken-β actin (CBA) promoter. GFP expression indicate that AAV2/5 is less efficiently transducing primary neurons than AAV2/6 and AAV2/DJ whereas no major differences were observed between AAV2/6 and AAV2/DJ, suggesting that these serotypes are particularly suitable for *in vitro* studies. (**F**) Transduction efficiency of an AAV2/5 expressing GFP under the CBA promoter was evaluated in the mouse brain. A total of 9.5x10⁷ viral genomes (Vg) were injected in the mouse striatum. Direct fluorescence imaging shows a strong GFP signal spread over a large region of the striatum.

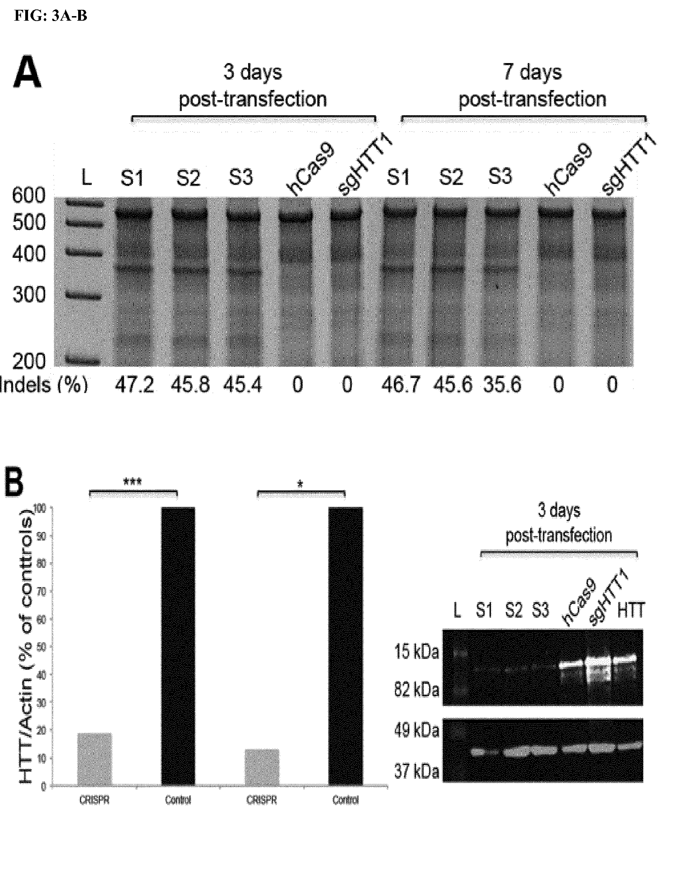

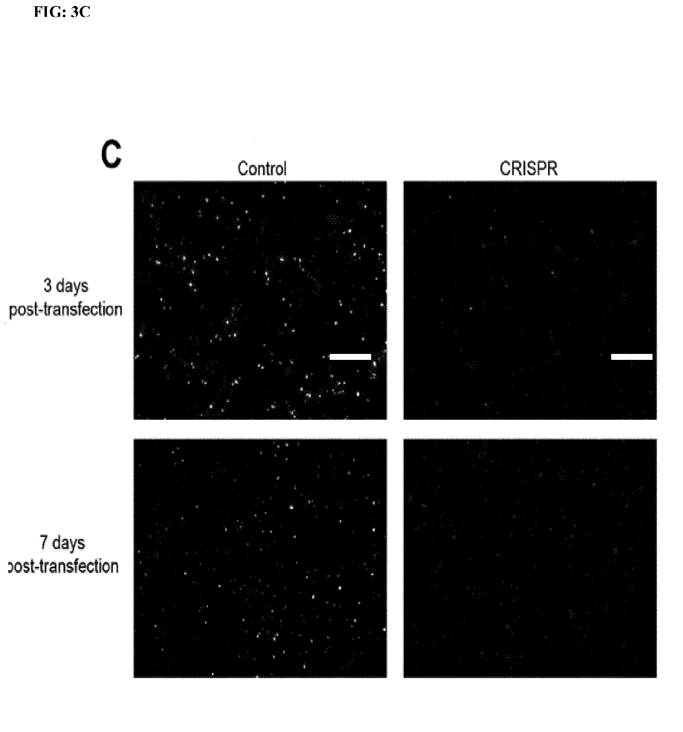

Figure 3: Human *HTT* editing in WT HEK-293T cells. (**A**) Plasmids coding for hCas9 and sgHTT1 were transfected in HEK-293T cells for a period of 3 and 7 days. Genomic DNA was extracted for surveyor mutation detection analysis. Results show a mean of 45% of DSB formation at the *HTT* locus at both time points. S1-3: independent triplicates transfected with plasmids hCas9 and sgHTT1. The hCas9: control sample transfected with hCas9 and an empty plasmid. The sgHTT1: control sample transfected with sgHTT1 and an empty plasmid. (**B**) Plasmids coding for hCas9, sgHTT1 and the first 171 amino-acids of the human HTT with 82 CAG fused to the GFP (Htt171-82Q-GFP) were transfected in HEK-293T cells for 3 and 7 days. Total proteins were extracted and the amount of mutant HTT was evaluated by western blot with an anti-HTT antibody. Results display a strong loss of mutant HTT in samples treated with hCas9 and the sgHTT1 when compared with control samples. Actin was used as control protein for normalization. Results are presented as mean ± SEM. * p < 0.05, *** p < 0.001. (**C**) Analysis 3 and 7 days post-transfection indicates that control cells (Control panels) transfected with hCas9, Htt171-82Q-GFP and an sgRNA (no homology for either the target plasmid) have a high number of GFP-positive aggregates whereas HEK-293T cells transfected with hCas9, sgHTT1 and Htt171-82Q-GFP (CRISPR panels) have a reduced number GFP-positive aggregates of less intense GFP-signal. Scale bar = 200µm.

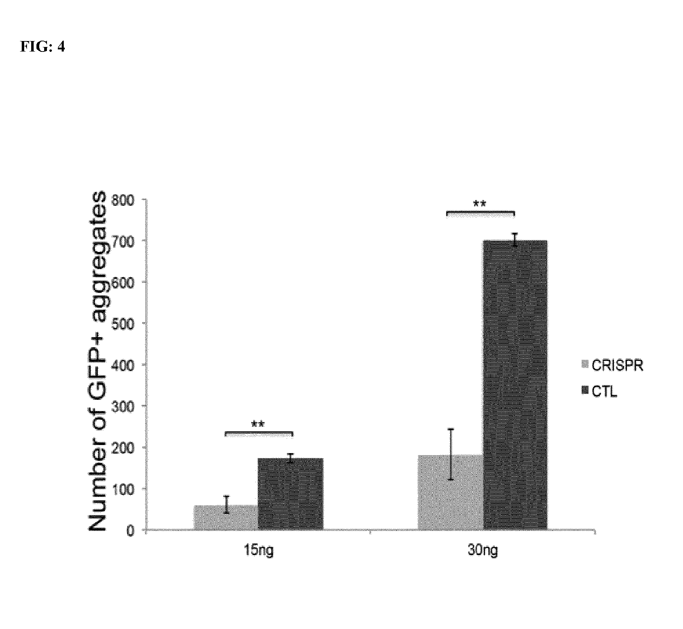

Figure 4: Evaluation of CRISPR efficiency in rat primary neuronal cells. The number of GFP-positive aggregates were counted in both treated and control groups. Quantifications show that number of aggregates is reduced in treated cells when compared to control samples. Microscopic acquisitions confirm the loss of aggregates. Scale bar = 50µm. Results are expressed as mean ± SEM. ** p < 0.01.

### BRIEF DESCRIPTION OF THE SEQUENCES

SEQ ID NO: 1 corresponds to the 82 nucleotides of the chimeric tracrRNA containing the poly-T signal: 5'-GTTTTAGAGCTAGAAATAGCAAGTTAAAATAAGGCTAGTCCGTTATCAACTTGAA AAAGTGGCACCGAGTCGGTGCTTTTTT -3'
SEQ ID NO: 2 corresponds to the 20 nucleotides of the sgTARGET: 5'- GACCCTGGAAAAGCTGATGA -3'
SEQ ID NO: 3 corresponds to the 20 nucleotides of the sgGFP: 5'-GAAGTTCGAGGGCGACACCC -3'
SEQ ID NO: 4 corresponds to the 20 nucleotides of the sgHTT1, which is conserved between humans, *Macaca Fascicularis* and *Mus Musculus* with one mismatch at position 1 (G/A) : 5'- GACCCTGGAAAAGCTGATGA -3'
SEQ ID NO: 5 corresponds to the 20 nucleotides of the sgHTT2, which is fully conserved between humans, *Macaca Fascicularis*, *Rattus Norvegicus* and *Mus Musculus*: 5'- GTGGATGACATAATGCTTTT -3'
SEQ ID NO: 6 corresponds to the 20 nucleotides of the sgHTT3, which is conserved between humans, *Macaca Fascicularis*, *Rattus Norvegicus* and *Mus Musculus* with one mismatch at position 4 (G/A): 5'- GGAGCCGCTGCACCGACCGT -3'
SEQ ID NO: 7 corresponds to the 20 nucleotides of the sgHTT4, which is human specific with SNP rs13102260 at position 20 for allele-specific approaches: 5'- TGGGACGCAAGGCGCCGTGG -3'
SEQ ID NO: 8 corresponds to the human codon-optimized Cas9 fused to one nuclear localization signal (sequence: https://www.addgene.org/44185/, (15).
SEQ ID NO: 9 corresponds the HR sequence used in the example 2 containing the end of the mCherry, the corrected target site and the GFP sequence: 
5’-CTGCCCGGCGCCTACAACGTCAACATCAAGTTGGACATCACCTCCCACAACGA
GGACTACACCATCGTGGAACAGTACGAACGCGCCGAGGGCCGCCACTCCACCGG
CGGCATGGACGAGCTGTACAAGGGTACCAGCGCCGCGTCGAGAGATGGGGGA
GGCTAACAGAAGTGTACGCGTTGGCTGCTGTCAGGGGTACCATGGACGCGTAGA
CAAGGTGAGGAACCTAGCTCAGTCATGCACCGGGCTGCAGATGAGCAAGGGCGA
GGAGCTGTTCACCGGGGTGGTGCCCATCCTGGTCGAGCTGGACGGCGACGTAAAC
GGCCACAAGTTCAGCGTGTCCGGCGAGGGCGAGGGCGATGCCACCTACGGCAAG
CTGACCCTGAAGTTCATCTGCACCACCGGCAAGCTGCCCGTGCCCTGGCCCACCC
TCGTGACCACCCTGACCTACGGCGTGCAGTGCTTCAGCCGCTACCCCGACCACAT
GAAGCAGCACGACTTCTTCAAGTCCGCCATGCCCGAAGGCTACGTCCAGGAGCGC
ACCATCTTCTTCAAGGACGACGGCAACTACAAGACCCGCGCCGAGGTGAAGTTCG
AGGGCGACACCCTGGTGAACCGCATCGAGCTGAAGGGCATCGACTTCAAGGAGG
ACGGCAACATCCTGGGGCACAAGCTGGAGTACAACTACAACAGCCACAACGTCT
ATATCATGGCCGACAAGCAGAAGAACGGCATCAAGGCCAACTTCAAGATCCGCC
ACAACATCGAGGACGGCAGCGTGCAGCTCGCCGACCACTACCAGCAGAACACCC
CCATCGGCGACGGCCCCGTGCTGCTGCCCGACAACCACTACCTGAGCACCCAGTC
CGCCCTGAGCAAAGACCCCAACGAGAAGCGCGATCACATGGTCCTGCTGGAGTT
CGTGACCGCCGCCGGGATCACTCACGGCATGGACGAGCTGTACAAGTGA -3’
SEQ ID NO: 10 corresponds to the SNP rs13102260. Species: human HTT SNP.
SEQ ID NO: 11 corresponds to the SNP rs61792465. Species: human HTT SNP.
SEQ ID NO: 12 corresponds to the SNP rs76533208. Species: human HTT SNP.
SEQ ID NO: 13 corresponds to the SNP rs112396951. Species: human HTT SNP.
SEQ ID NO: 14 corresponds to all the 1132 sequences followed by a NGG/NAG PAM on both strands +/- 4kb from the HTT ATG.

### DETAILED DESCRIPTION OF THE INVENTION

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise" is generally used in the sense of include, that is to say permitting the presence of one or more features or components.

As used in the specification and claims, the singular forms "a", "an" and "the" include plural references unless the context clearly dictates otherwise.

As used herein the terms "subject" or "patient" are well-recognized in the art, and, are used interchangeably herein to refer to a mammal, including dog, cat, rat, mouse, monkey, cow, horse, goat, sheep, pig, camel, and, most preferably, a human. In some embodiments, the subject is a subject in need of treatment or a subject with a disease or disorder. However, in other embodiments, the subject can be a normal subject. The term does not denote a particular age or sex. Thus, adult and newborn subjects, whether male or female, are intended to be covered.

The term "an effective amount" refers to an amount necessary to obtain a physiological effect. The physiological effect may be achieved by one application dose or by repeated applications. The dosage administered may, of course, vary depending upon known factors, such as the physiological characteristics of the particular composition; the age, health and weight of the subject; the nature and extent of the symptoms; the kind of concurrent treatment; the frequency of treatment; and the effect desired and can be adjusted by a person skilled in the art.

The terms "nucleic acid," "polynucleotide," and "oligonucleotide" are used interchangeably and refer to a deoxyribonucleotide or ribonucleotide polymer, in linear or circular conformation, and in either single- or double-stranded form. For the purposes of the present disclosure, these terms are not to be construed as limiting with respect to the length of a polymer. The terms can encompass known analogues of natural nucleotides, as well as nucleotides that are modified in the base, sugar and/or phosphate moieties (e.g. phosphorothioate backbones). In general, an analogue of a particular nucleotide has the same base-pairing specificity; i.e., an analogue of A will base-pair with T.

The terms "polypeptide," "peptide" and "protein" are used interchangeably to refer to a polymer of amino acid residues. The term also applies to amino acid polymers in which one or more amino acids are chemical analogues or modified derivatives of a corresponding naturally-occurring amino acids.

"Recombination" refers to a process of exchange of genetic information between two polynucleotides. For the purposes of this disclosure, "homologous recombination (HR)" refers to the specialized form of such exchange that takes place, for example, during repair of double-strand breaks in cells via homology-directed repair mechanisms. This process requires nucleotide sequence homology, uses a "donor" molecule to template repair of a "target" molecule (i.e. the one that experienced the double-strand break), and is variously known as "non- crossover gene conversion" or "short tract gene conversion," because it leads to the transfer of genetic information from the donor to the target. Without wishing to be bound by any particular theory, such transfer can involve mismatch correction of heteroduplex DNA that forms between the broken target and the donor, and/or "synthesis-dependent strand annealing," in which the donor is used to resynthesize genetic information that will become part of the target, and/or related processes. Such specialized HR often results in an alteration of the sequence of the target molecule such that part or all of the sequence of the donor polynucleotide is incorporated into the target polynucleotide.

A "reporter gene" or "reporter sequence" refers to any sequence that produces a protein product that is easily measured, preferably although not necessarily in a routine assay. Suitable reporter genes include, but are not limited to, sequences encoding proteins that mediate antibiotic resistance (e.g., ampicillin resistance, neomycin resistance, G418 resistance, puromycin resistance), sequences encoding colored or fluorescent or luminescent proteins (e.g., green fluorescent protein, enhanced green fluorescent protein, red fluorescent protein, luciferase), and proteins which mediate enhanced cell growth and/or gene amplification (e.g., dihydrofolate reductase). Epitope tags include, for example, one or more copies of FLAG, His, myc, Tap, HA or any detectable amino acid sequence. "Expression tags" include sequences that encode reporters that may be operably linked to a desired gene sequence in order to monitor expression of the gene of interest.

"The CRISPR" (Clustered Regularly Interspaced Short Palindromic Repeats)/Cas (CRISPR Associated) nuclease system is a recently engineered nuclease system based on a bacterial system that can be used for genome engineering. It is based on part of the adaptive immune response of many bacteria and archea. When a virus or plasmid invades a bacterium, segments of the invader's DNA are converted into CRISPR RNAs (crRNA) by the 'immune' response. This crRNA then associates, through a region of partial complementarity, with another type of RNA called tracrRNA to guide the Cas9 nuclease to a region homologous to the crRNA in the target DNA called a "protospacer." Cas9 cleaves the DNA to generate blunt ends at the DSB at sites specified by a 20-nucleotide guide sequence contained within the crRNA transcript. Cas9 requires both the crRNA and the tracrRNA for site specific DNA recognition and cleavage. This system has now been engineered such that the crRNA and tracrRNA can be combined into one molecule (the "single guide RNA"), and the crRNA equivalent portion of the single guide RNA can be engineered to guide the Cas9 nuclease to target any desired sequence (see Jineket al (2012) Science 337, p. 816-821, Jineket al, (2013), eLife 2:e00471, and David Segal, (2013) eLife 2:e00563). Thus, the CRISPR/Cas system can be engineered to create a DSB at a desired target in a genome, and repair of the DSB can be influenced by the use of repair inhibitors to cause an increase in error prone repair.

As used herein the term "selectivity of an sgRNA" relates to the ability for an sgRNA sequence and Cas9 complex, to recognize a particular allele of a target gene (i.e. mutant vs WT allele of *HTT*).

The term "specificity of an sgRNA" relates to the ability for a given sgRNA sequence and Cas9 complex to recognize a given target i.e., the *HTT* gene.

The term "haplotype" relates to the ordered, linear combination of polymorphisms (e.g., SNPs) in the sequence of each form of a gene (on individual chromosomes) that exists in the population.
The term "small insertions-deletions (indels)" relates to small insertion or deletions in genomic DNA due to cellular DNA repair following DSB by non-homologous end joining (NHEJ).

The term "allele" relates to a particular form of a genetic locus, distinguished from other forms by its specific nucleotide sequence.

A "Single Nucleotide Polymorphism" (SNP) refers to a single base (nucleotide) difference in a specific location in the DNA sequence among individuals in a population. A subset of SNPs gives rise to changes in the encoded amino acid sequence; these are referred to as coding SNPs, or cSNPs.

The term "allele-specific" when used in reference to nucleic acid sequences, such as oligonucleotides, primers, sgRNA, means that a particular position of the nucleic acid sequence is complementary with an allele of a target polynucleotide sequence. Allele-specific sgRNA are capable of discriminating between different alleles of a target polynucleotide.

The term "vector", as used herein, refers to a DNA or RNA molecule such as a plasmid, virus or other vehicle, which contains one or more heterologous or recombinant DNA sequences and is designed for transfer between different host cells. The terms "expression vector" and "gene therapy vector" refer to any vector that is effective to incorporate and express heterologous DNA fragments in a cell. A cloning or expression vector may comprise additional elements, for example, post-transcriptional regulatory elements. Any suitable vector can be employed that is effective for introduction of nucleic acids into cells, e.g. a viral vector. The terms "heterologous DNA" refer to a "heterologous coding or non-coding sequence" or a "transgene".

It is one object of the invention to provide a kit for the treatment of Huntington's disease (HD) for allele or non-allele-specific huntingtin (*HTT*) gene editing or repair comprising:
a gene delivery vector consisting of at least one viral vector selected among adeno-associated vectors (AAV) and/or lentiviral vectors (LV);
a Cas9 being human codon-optimized ;
an artificial single guide RNA (sgRNA) having a total size from 63-115 nucleotides and preferably 102 nucleotides comprising a crRNA sequence of 15-30 nucleotides, preferably 20 nt, recognizing the *HTT* gene and capable of targeting around the ATG +/- 4 kb of the *HTT* gene wherein said sgRNA further comprises a tracrRNA sequence of 48-85 nucleotides long, preferably 82 nt long.

Preferably the gene delivery vector contains various expression cassettes comprising promoters and/or miRNA-regulated system so as to modulate transgene expression.

In a preferred mode of the invention, the AAV is a mutant vector and preferably the AAV-DJ.

In another embodiment, of the invention, the Cas9 is mutated to improve the efficiency and safety. Preferably said mutated Cas9 is a Cas9 nickase.

In another embodiment of the invention the gene delivery vector consist in a single vector or separated vectors.

According to a preferred mode of the invention, the kit comprises multiple transcriptionally independent sgRNA targeting the *HTT* gene.

In particular, said artificial sgRNA comprises a sequence (crRNA) recognizing a target site of 15-30 nucleotides, preferably 20 nt, recognizing the *HTT* gene is human-specific or a conserved sequence between human, rodent and primates with a maximum tolerated number of mismatches between sgRNA and *HTT* gene of 3 in the first 8 nucleotides of the sgRNA.

According to a preferred mode, said artificial crRNA sequence of 15-30 nucleotides is encoded by the sequences selected among the group of SEQ ID NO: 2-7 and/or biological active fragments, variants or mutants thereof being able to recognize the *HTT* gene and capable of targeting around the ATG +/- 4 kb of the *HTT* gene.

In another embodiment of the invention, the kit further comprises an exogenous DNA template for homologous recombination (HR) of human *HTT.*

It is another object of the invention to provide a kit for the treatment of Huntington's disease, for use in a method for non-allele-specific *HTT* inactivation of the human (or other species having a conserved sequences) *HTT* WT (wild type) and mutant alleles.

Another object of the invention is to provide a kit for the treatment of Huntington's disease, for use in a method for non-allele-specific *HTT* gene repair based on HR with a DNA template containing a WT *HTT* sequence.

A further object of the invention is to provide a kit for the treatment of Huntington's disease, for use in a method for allele-specific *HTT* inactivation of the human mutant *HTT* gene characterized in that the sgRNA of said kit is capable of recognizing SNP sequences located around the ATG of the *HTT* gene of +/- 4 kb and with a high frequency of heterozygosity in the human population.

The invention also provide a kit for the treatment of Huntington's disease, for use in a method for mutant *HTT* gene repair based on HR with a DNA template containing a WT *HTT* sequence characterized in that the sgRNA of said kit is capable of recognizing SNP sequences located around the ATG of the *HTT* gene of +/- 4 kb and with a high frequency of heterozygosity in the human population.

Applicants propose to use gene editing with the CRISPR system approach for HD. It concerns a new therapy for a fatal neurodegenerative disorder with no available treatment. This represents the first attempt of in vivo gene repair for a CNS disorder.

According Applicants strategy is not based on repressors to inhibit *mHTT* expression but will adress the possibility to perform gene editing to inactivate mutant HTT and DNA repair restore WT HTT expression using DNA-targeted nucleases. In particular, one will use the recently described CRISPR system to induce *mHTT* double-strand break for permanent *mHTT* knock-out or repair.

One important prerequisite for the development of in vivo gene repair strategy in the CNS is an efficient delivery system. Applicants have integrated the CRISPR system in lentiviral vectors (LV expressing Cas9 and the sgRNA) and adeno-associated virus (AAV containing the donor DNA for HR), which have been widely used in vitro but also for in vivo gene delivery in the CNS.

As a first step toward the establishment of an in vitro gene editing system for mHTT, Applicants performed experiments with reporter genes in HEK 293T cells. For this, a sequence containing the mCherry fluorescent protein followed by the CRIPR target sequence containing a STOP codon and the GFP gene was integrated in the genome of HEK 293T cells using LV. mCherry-positive cells were then infected with LV expressing Cas9 and an sgRNA recognizing the target sequence close to the stop codon. As controls, Applicants infected cells with Cas9 or sgRNA alone. The potency of DNA DSB appearance and NHEJ was quantified with the surveyor nuclease assay and specific restriction-length fragment polymorphisms (RFLP). Results at the DNA levels show up to 6145% of DSB, 2 weeks post-infection. Using Western blots, we observed the fusion mCherry-GFP protein after NHEJ formation. Additionally, 3 days post-transfection of HEK-mCherry-GFP cells with a plasmid containing the sequence for homologous recombination without a stop codon, Applicants observed around 27% of HR. Finally, Applicants infected rat primary neuronal cultures with LV expressing Cas9, eGFP and Tomato-sgRNA targeting the eGFP sequence. Applicants observed strong eGFP loss in neurons expressing Tomato fluorescent protein, 3 weeks post-infection. This shows that Applicants' CRISPR system efficiently knockout eGFP following DSB formation in primary cells.

Applicants evaluated the efficiency of the CRISPR system in the mouse brain to disrupt eGFP sequence. Applicants have injected LV encoding Cas9 ans sgGFP sequences into the striatum of double-transgenic mice BAC Drd1/Drd2-eGFP, expressing eGFP in both D1 and D2 neurons of the striatum. No toxicity or adverse effects were observed up to 4 weeks post-infection in neurons. In addition, a strong loss of GFP signal was found 3 and 4 weeks post-infection in the infected area. Importantly, eGFP-negative neurons are still expressing the GABAergic marker DARPP32, confirming that eGFP loss is due to gene disruption and not major neuronal dysfunction.

The Applicants use gene editing with the CRISPR system as therapeutic strategy for HD. One important prerequisite for the development of *in vivo* gene editing/repair strategy in the CNS is an efficient delivery system. Applicants use reporter genes to develop and validate VSV-G pseudotyped LV (but other envelopes might be considered) and AAV vectors (AAV2/5, AAV2/6 and AAV-DJ, but other serotypes or chimeric AAV variants might be considered, including AAV1, 9, 10) (16). The AAV-DJ has been shown to increase spontaneous HR efficiency (17) and combined with the CRISPR system is increasing *HTT* editing efficiency. These gene transfer systems are suitable for *in vitro* and *in vivo* delivery (intraparenchymal or intraventricular administrations) of the CRISPR system (Examples 2-5, Figures 2-5). To optimize *HTT* gene editing, various vectors and expression cassettes were produced and tested (choice of the promoters driving the expression of the transgenes, ratio between Cas9 and sgRNA, integration of multiple sgRNA, integration of the CRISPR system in a single vector or in two separate vectors).

To block mutant *HTT* (*mHTT*) expression, Applicants did not use ZFN-repressors or TALEN-repressors (WO 2013/130824) but did disrupt the *HTT* gene (non-allele or allele-specific targeting) using DNA-targeted nucleases. In particular, Applicants use the recently described CRISPR system to induce *mHTT* double-strand break (DSB) around the transcription start site (TSS) or translation initiation codon (ATG) of the *HTT* gene for permanent and irreversible *mHTT* inactivation (Examples 3-4, Figures 3-4). Both DNA strand (sense or anti-sense) are targeted by the sgRNA. For allele-specific inactivation, Applicants selected single-nucleotide polymorphisms (SNP) located close to the TSS/ATG of the *HTT* gene (+/- 24 kb from the TSS) and designed sgRNA targeting the disease isoform of heterozygous single-nucleotide polymorphisms (SNP). Transfection of human embryonic kidney cells (HEK 293T cells) with a plasmid expressing a fragment of the human *mHTT* (first 171 amino acids with 82 CAG repeats) fused to the green fluorescent protein (GFP) reporter gene was used to evaluate double-stranded breaks (DSB) in target *HTT* DNA and non-homologous end joining (NHEJ) which is leading to mall insertion-deletion formation (indels). In 2/3 of the cases, indels modify the reading frame of the HTT-GFP and this lead to a loss of expression of the fluorescent protein (GFP). Surveyor, western blots and cell imaging were used to confirm and quantify DSB formation (Example 3-4, Figure 3-4).

The feasibility and efficacy of the approach was further validated in post-mitotic cells primary neuronal cultures with the GFP reporter gene. A strong GFP loss was observed 3 weeks post-infection of rat striatal cultures with Cas9/sgGFP suggesting that the CRISPR system efficiently knock-out GFP following DSB formation in primary post-mitotic cells (Example 3, Figure 3).

The capacity to perform gene editing *in vivo* was first assessed in transgenic mice expressing the GFP reporter gene in GABAergic neurons of the striatum (D1/D2-GFP mice). The instrastriatal injection of viral vectors encoding the Cas9 and / or sgRNA targeting the GFP reporter gene was not associated with significant toxicity (Example 5). Confocal acquisitions reveal a strong loss of GFP signal in cells expressing the sgGFP (Example 5). Applicants further studied the functionality of neurons with disrupted GFP expression with the marker of the GABAergic neurons, DARPP-32. In the infected area, Applicants observed that eGFP-negative cells are DARPP32-positive (Example 5). This demonstrates that GFP loss is the result of GFP disruption by the CRISPR system and not due to a global neuronal dysfunction or cells toxicity/death. In addition, no adverse effects (based in immunohistochemical analysis with Iba1, CD11b, GFAP markers, data not shown) were observed up to 3 weeks post-infection. These results demonstrate the feasibility of genetic modifications both *in vitro* and in the CNS of adult rodent with the CRISPR system.

Species-specific or sgRNA targeting conserved region of the *HTT* gene in human but also rodent and non-human primates were selected. The later one will greatly facilitate the pre-clinical validation of *HTT* gene editing in rodent and large animal models (primates) (SEQ ID NO: 7).

As a second strategy for the allele-specific gene editing of mutant *HTT* gene, Applicants use two sgRNAs, one targeting the TSS/ATG or a SNP located close to the TSS/ATG of the *HTT* gene and a second sgRNA targeting the disease isoform of heterozygous single-nucleotide polymorphisms (SNP) located after the CAG expansion and preferentially in an intron (Figure 1B). This leads to the deletion of the entire exon 1 containing the ATG codon of the *HTT.* Finally a non allele-specific or allele-specific DNA repair strategy was developed. An sgRNA targeting intron 1 of the *HTT* gene has been used to induce DSB and a donor sequence containing the WT human *HTT* exon1 and flanking sequences was used to induce homologous recombination. For the allele-specific repair of *mHTT,* Applicants used sgRNA targeting the disease isoform of heterozygous single-nucleotide polymorphisms (SNP) located after the CAG expansion and preferentially in an intron (Figure 1B).

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency are intended to be embraced therein.

Various references are cited throughout this specification, each of which is incorporated herein by reference in its entirety.

The foregoing description will be more fully understood with reference to the following Examples. Such Examples, are, however, exemplary of methods of practicing the present invention and are not intended to limit the scope of the invention.

### Examples

### Example 1: Development of a viral-based delivery system for CRISPR

### Material and methods

### Plasmid constriction

The pcDNA3.3-TOPO-CMV-hCas9 plasmid contains the human codon-optimized Cas9 (hCas9) sequence fused to a nuclear localization signal expressed under the CMV promoter (Addgene, Cambridge MA, USA). The hCas9 gene was excised from the plasmid using PmeI and NcoI (Roche Diagnostics GmbH, Mannheim, Germany) restriction sites and inserted into a pENTR-dual plasmid (Invitrogen, Life Technologies, Regensburg, Germany) digested with NcoI and EcoRV (Roche Diagnostics GmbH, Mannheim, Germany) (pENTR-dual-hCas9). Finally, a site-specific recombination was performed with the gateway system (Invitrogen, Regensburg, Germany; (5)) and the destination vector SIN-cPPT-PGK-RFA-WPRE and SIN-cPPT-CMV-RFA-WPRE (16) to produce the SIN-cPPT-PGK-hCas9-WPRE and SIN-cPPT-CMV-hCas9-WPRE. The sgRNA are expressed under the control of the HI polymerase III promoter. The H1-sgTARGET (targeting the mCherry-target-GFP sequence, SEQ ID NO 2; Figure 1A) was synthesized and cloned in pDONR221 vector (GeneArt, Invitrogen, Life Technologies, Regensburg, Germany). The sgRNA targeting the GFP (sgGFP) was synthesized in a pMK-T vector (GeneArt, Invitrogen, Life Technologies, Regensburg, Germany) and transferred in pDONR221 (pDONR221-H1-sgGFP). Finally, sgRNA targeting the huntingtin (sgHTT1-sgHTTn; SEQ ID NO 4-7) were synthesized as phosphorylated oligonucleotides (MicrosynthAG, Balgachm, Switzerland) and annealed in a thermocycler (BioLabo, Chatel-Saint-Denis, Switzerland). The resulting double-stranded DNA was digested by using BglII and NdeI restriction sites (Roche Diagnostics GmbH, Mannheim, Germany) and inserted into the pDONR221-H1-sgRNA digested by BglII and NdeI (pDONR221-H1-sgHTT). A gateway reaction was used to insert each sgRNA in the transfer vectors for LV production (SIN-H1-sgRNA-WPRE and SIN-cPPT-PGK-tdTomato-WPRE-LTR-TREtight-H1-sgRNA) (5, 16, 17). Plasmid coding for the mCherry-target-GFP sequence was designed, synthesized and cloned in pDONR221 vector (GeneArt, Invitrogen, Life Technologies, Regensburg, Germany) (pDONR221-mCherry-target-GFP) (Figure 1A). A LR clonase (Invitrogen, Life Technologies, Regensburg, Germany) was used to insert the target sequence into a transfer vector for LV production (SIN-cPPT-PGK-mCherry-target-GFP-WPRE). The DNA template for HR with the mCherry-target-GFP reporter system (Figure 1A) was synthesized (GeneArt, Invitrogen, Life Technologies, Regensburg, Germany) and cloned into the pDONR221 plasmid (pDONR221-template). Finally it was transferred into the pAAV2ss-ITR-DEST-RFA-bGHpoly-ITR plasmid by LR clonase reaction (kindly provided by Dr. A. Bemelmans, Fontenay-aux-Roses, France) to produce the AAV2-ITR-template-bGHpolyA-ITR.

To evaluate NHEJ with sgRNA targeting the human *HTT,* a plasmid encoding the mutant *HTT* (first 171 amino acids of the human HTT with 82 CAG repeats; (18) was fused to the eGFP (Clontech, Saint-Germain-en-Laye, France) containing 5 copies of myc tag epitope (EQKLISEEDL). This fusion construct was cloned in a third generation LV transfer vector (19) under a CMV promoter (pRRL-cPPT-CMV-Htt171-82Q-5xMyc-GFP-WPRE). To identify infected cells *in vivo,* a LV transfer vector encoding the red monomer fluorescent protein mCherry was used (SIN-cPPT-PGK-mCherry-WPRE).

### Cell culture

HEK-293T cells (LGC, Wesel, Germany) were cultured in DMEM-Glutamax supplemented with 10% FBS and 1% Pennicilin/Streptomycin (Gibco, Life Technologies, Zug, Switzerland). Cells were passed every three days using Tryspsin for dissociation (Gibco, Life Technologies, Zug, Switzerland). For transfection experiments, 300'000 cells were plated in corresponding wells (6-wells, 24-wells or 48-wells; Vitaris, Baar, Switzerland) the day before transfection. Transfections were done using calcium-phosphate method. Up to 4µg total of plasmids for 9.5cm2 plate (6 well) were mixed in equal amount of H₂O and CaCl₂ and then added drop by drop in HEPES (Sigma-Aldrich, Buchs, Switzerland, ratio plasmid-H₂O-CaCl₂:HEPES = 1:1). Mixture was incubated at RT for 5 minutes and then added to the corresponding cultures drop by drop. Medium was completely changed 6 hours post-transfection.

### LV production and titration

LV were produced in HEK-293T cells with the four-plasmid system, as described previously (17). The HIV-1 vectors were pseudotyped with the VSV-G envelope and concentrated by ultracentrifugation and resuspended in phosphate-buffered saline (PBS, Gibco, Life Technologies, Zug, Switzerland) with 1% bovine serum albumin (BSA, Sigma-Aldrich, Buchs, Switzerland). The viral particle content of each batch was determined by p24 antigen enzyme-linked immunosorbent assay (p24 ELISA, RETROtek; Kampenhout, Belgium). The stocks were finally stored at -80°C until use.

### AAV production

The AAV recombinant genome contains the transgene under the control of mammalian promoters, the WPRE, and the bovine hormone (bGH) gene polyadenylation signal, flanked by AAV2 ITRs. This expression cassette was encapsidated in an AAV-5, 6, DJ capsid as described previously (20-22). For this, the plasmids coding for the transgene, the capsid and Ad helper were transfected by calcium phosphate precipitation in HEK-293T cells. These cells encode the E1 region of the Ad5 genome. Cells and supernatant were harvested 3 days post-transfection and centrifuged at 300 g for 10 minutes at 4°C. Supernatant was supplemented with 8% PEG (Sigma-Aldrich, Buchs, Switzerland, 2.5M NaCl, Merck, Nottingham, UK) and was kept at 4°C for at least 2 hours. Cell pellets were pooled and incubated in lysis buffer (NaCl 0.15M, Merck, Nottingham, UK, Tris-HCl 50mM, pH 8.5, Sigma-Aldrich, Buchs, Switzerland) for 3 consecutive freeze/thaw cycles (30 minutes in dry ice/ethanol followed by 30 minutes at 37°C). PEG-containing supernatant was centrifuged at 4'000 g for 20 minutes at 4°C after the 2 hours incubation and supernatant was discarded. Cell lysate was added to the pellets and the mixture was incubated at 37°C for 1 hour to ensure full homogenization of pellets. Lysate was then treated with benzonase (0.15 units, Sigma-Aldrich, Buchs, Switzerland) in filtered MgCl2 1M at 37°C for 30 minutes (Sigma-Aldrich, Buchs, Switzerland). Treated lysate was clarified by centrifugation at 4'000 g for 20 minutes at 4°C. AAV were separated using iodixanol (AxonLab, Le Mont sur Lausanne, Switzerland) gradient ultracentrifugation at 59'000 rpm (70Ti rotor, Beckman-Coulter, Nyon, Switzerland) for 90 minutes at 20°C as previously described (Grieger et al 2006). Phase containing AAV was harvested and loaded on an Amicon Ultra-15 PL 100 (Millipore, Zug, Switzerland) with 0.001% Pluronic F68 D-PBS (Gibco, Life Technologies, Zug, Switzerland) for iodixanol cleaning and viral particles concentration. Tubes were first centrifuged at 4'000 g at 4°C until the totality of solution has passed through the column. Two additional wash with 0.001% Pluronic F68 D-PBS were done and AAV were finally resuspended in 250-500µL 0.001% Pluronic F68 D-PBS. AAV were kept at -80°C until the use. Titration was performed by qPCR as previously described (23).

### Results

To validate and assess the efficacy of the CRISPR system, two sgRNAs targeting the mCherry-target-GFP (sgTARGET) and GFP (sgGFP) sequences were designed (Figure 1A). In addition, sgRNA targeting the human, rodent and primate *HTT* were produced (sgHTT) (Figure 1B). The majority of them are targeting the 5' region of the gene to ensure loss of mutant *HTT* expression or repair of the mutation. The DNA templates for HR were delivered with AAV to ensure high number of episomes in the nucleus. The sgRNA under the control of the HI promoter were cloned into the 3' LTR of SIN plasmids expressing reporter gene to allow visualization of infected cells (5). Functionality of plasmids containing reporter genes was evaluated by transient transfection in HEK-293T cells (data not shown).

### Example 2: CRISPR optimization for DSB and HR

### Material and methods

### Cell culture

HEK-293T cell culture was performed as described in the example 1. An LV-SIN-cPPT-mCherry-target-GFP-WPRE containing a target sequence and a GFP fluorescent protein (Figure 1A) was used to infect HEK-293T cells and produce populations suitable to evaluate DSB formation (HEK-TARGET). HEK-TARGET cells were infected with the various amount of viral particles diluted in culture medium. Up to 600ng p24 were used to infect 3x10⁵ HEK-293T cells in 9.5cm2 wells (6 well). Cells were passed every 7 days after infection and medium was changed every 3 days.

### FACS

HEK-TARGET cells were harvested and processed for FACS analysis 3-5 weeks post-infection. The cells were washed and resuspended in small volume of sterile PBS to ensure high cell concentration (1-10x10⁶/ml). For each sample, 50'000 events were analyzed. mCherry-positive cells were sorted with a Beckman-Coulter system and were grown on 96-well plates. Based on mCherry fluorescence, four distinct cell populations were isolated: cells with low copy number of target sequence (populations 1 and 10) and cells with high copy number of target sequence (populations 50 and 100).

### qPCR

HEK-TARGET cells were passed and resuspended in PBS. Genomic DNA (gDNA) was extracted from 1.10⁶ cells with Trizol Reagent and resuspended at a concentration of 50 ng/µL in DNAse-free water. Provirus copy number was determined by SYBRgreen qPCR (KapaBiosystems, Le Mont sur Lausanne, Switzerland) and the primers HIV-1F-TGTGTGCCCGTCTGTTGTTGT and HIV-2R- GAGTCCTGCGTCGAGAGAGC. qPCR with primers targeting the endogenous human *HTT* were used as genomic standard (HTT-21F- ATGGACGGCCGCTCAGGTTCT, HTT-68R- GCTCAGCACCGGGGCAATGAA).. Serial dilutions of gDNA from each population were performed and compared to the standard curve of HTT. Provirus number per cell was quantified based on the Lenti-X provirus quantitation kit (Clontech, Saint-Germain-en-Laye, France).

### Primary neuronal cultures

Timed-pregnant Sprague-Dawley rats were killed by CO₂ inhalation and E17 embryos were collected in Petri dish containing HBSS. Striatum were dissected in corresponding medium containing H₂O, Na₂SO₄ 1M, K₂SO₄ 0.5M, MgCl₂ 1M, CaCl₂ 1M, HEPES 1M pH 7.3-7.4, Glucose 2.5M, Phenol red 0.5%, NaOH IN pH 7.4 (Sigma-Aldrich, Buchs, Switzerland) and Ky/Mg (1mM acide kynurenique/ 10mM MgCl₂, Sigma-Aldrich, Buchs, Switzerland) and kept on ice in a 15mL Falcon. Dissection medium was removed and structures were enzymatically dissociated in dissection medium containing 1 mg/mL L-cysteine and 0.125mg/mL papain (Sigma-Aldrich, Buchs, Switzerland). Mixture was incubated at 37°C for 10 minutes, solution was removed and the operation was repeated a second time. After the final incubation, all the papain/cysteine solution was removed and lysates were washed 3 times in dissection medium. Washed lysates were then incubated in dissection medium containing 10mg/mL of trypsin inhibitor containing solution for 7 minutes 30 seconds at 37°C. Lysates were then washed 3 times in dissection medium and 3 times in Opti-MEM-Glucose 2.5M (Gibco, Life Technologies, Zug, Switzerland) solution after final dissociation in Opti-MEM-Glucose 2.5M using Paster pipettes. Fully dissociated cells were pooled in the same tube and counted. The cells were platted at a density of 1,25 x 10⁴ cells per cm2 in multi-wells dishes in Opti-MEM-Glucose 2.5M medium. Medium was replaced 4 hours after platting by Neurobasal supplemented with 2% B27, 1% penicillin/Streptamycin and 1% Glutamax (Gibco, Life Technologies, Zug, Switzerland).

Different AAV serotypes were compared to determine the best delivery system for the DNA template *in vitro.* One day post-seeding of HEK-293T cells, 1x10⁷ Vg of AAV2/5, AAV2/6 and 5.5x10⁶ Vg AAV2/DJ-ITR-CBA-GFP-WPRE-ITR were incubated on cells. The cultures were fixed 2 weeks post-infection in 4% paraformaldehyde (Electron Microscopy Sciences, Hatfield) at room temperature (RT) for 10 minutes, washed 3 times in PBS and analyzed for direct GFP fluorescence.

### Stereotaxic surgery

Concentrated viral stocks were thawed on ice and resuspended by repeated pipetting. AAV were diluted in D-PBS 1x (Gibco, Life Technologies, Zug, Switzerland) to inject 9.5x 10⁷ Vg in 2 µL.

Mice were anesthetized by intraperitoneal injection of a mixture of 100 mg/kg ketamine (Ketasol, Graeub, Bern, Switzerland) and 10 mg/kg xylazine (Rompun, Bayer Health Care, Uznach, Switzerland). Suspensions of AAV were injected into the mouse striatum with a 34-gauge blunt-tip needle linked to a Hamilton syringe by a polyethylene catheter. Stereotaxic coordinates were: anteroposterior +1 mm from bregma; mediolateral +/-1.5 mm and dorsoventral -3.5 mm from the skull, with the tooth bar set at -3.3 mm. Mice received a total volume of 3 µl of the vector preparation, administered at a rate of 0.2 µl/min. At the end of injections, needles were left in place for 5 min before being slowly removed. The skin was sutured with 6-0 Prolene suture (B-Braun Mediacal SA, Sempach, Switzerland) and mice were allowed to recover on a heating mat.

### DNA, RNA and proteins extractions

DNA, RNA and proteins were extracted from the same sample with the Trizol reagent according to the manufacturer's protocol (Invitrogen, Life Technologies, Zug, Switzerland). Briefly, cells were washed in PBS (Gibco, Life Technologies, Zug, Switzerland) and lysed in Trizol. DNA and RNA were resuspended in RNAse-DNAse sterile water (Gibco, Life Technologies, Zug, Switzerland) whereas proteins were resuspended in 1% SDS, 10% Urea solution (Sigma-Aldrich, Buchs, Switzerland). RNA were kept at -80°C, proteins were kept at -20°C and DNA diluted to 100ng/µL were conserved at 4°C.

### Surveyor analysis

DSB formation was evaluated using the surveyor mutation detection kit, which detects mutations and polymorphisms in DNA (Transgenomic Inc, Glasgow, UK) (24). Briefly, forward (TARGET-IF: ACGGCGAGTTCATCTACAAGGTGAAGCTGC) and reverse oligonucleotides (TARGET-2R: TTGTACTCCAGCTTGTGCCCCAGGATGTTG) complementary to a region located around the target sequence in the genomic DNA (200 ng) are used with the Taq polymerase (Roche Diagnostics GmbH, Mannheim, Germany) to perform a PCR reaction. The concentration of the amplified fragment is evaluated by agarose gel electrophoresis using the GeneRuller 1kb DNA ladder (Thermo Scientific, Reinach, Switzerland). Four hundred nanograms of PCR product is incubated at 95°C in a thermocycler and subjected to the surveyor nuclease according to the manufacturer's protocol. Migration of the digested products is done on 10% TBE polyacrylamide precast gels (BioRad, Reinach, Switzerland). DNA is stained using SYBR gold (Molecular Probes, Life Technologies, Zug, Switzerland). The percentage of indels is determined by quantifying band intensities with Image J software (http://rsb.info.nih.gov/ij/, NIH, Bethesda) as described by Ran and collaborators (25).

### Restriction fragment length polymorphism (RFLP)

RFLP analysis exploits DNA variations and in particular restriction enzymes. The DNA sample is digested and the resulting restriction fragments are separated according to their lengths by gel electrophoresis. In the specific example described here, Applicants used 300-500ng of PCR product and digest it with the restriction enzyme NcoI for 2h at 37°C (New England Biolabs, Allschwill, Switzerland). The resulting products were analysed on a 10% TBE polyacrylamide precast gel (BioRad, Reinach, Switzerland) and stained in SYBR gold (Molecular Probes, Life Technologies, Zug, Switzerland). The Image J software was used to quantify the bands intensities and determine the percentage of RFLP (25).

### Western blot analysis

Two days after transfection, the 293T cells were harvested and lysed Trizol. Protein concentration was determined by the Bradford method (Thermo Scientific, Reinach, Switzerland). Twenty micrograms of proteins were run on 12% SDS-polyacrylamide gels, and transferred to nitrocellulose membranes in 0.025M Tris, 0.192M glycine, and 20% ethanol (26). The membranes were washed 3 x 10 minutes in PBS and blocked 1 hour in 33% LI-COR blocking buffer in 1% PBS-Tween20 (blocking, LI-COR, Bad Homburg, Germany) at RT under agitation. Primary antibodies were incubated in blocking solution overnight at 4°C under agitation. The following antibodies were used: anti-mCherry antibody (mCherry, 1/1000, SicGen, Lisboa, Portugal), goat anti-HTT antibody (HTT, 1/1000, SicGen, Lisboa, Portugal) and rabbit anti-Actin antibody (Actin, 1/1000, Abcam, Cambridge, UK). Membranes were rinsed 3 x 10 minutes in PBS-Tween20 and incubated with the following secondary antibodies in blocking for 1 hour at RT under agitation: donkey anti-goat IgG IRdye 800 (1/7000, LI-COR, Bad Homburg, Germany) and donkey anti-rabbit IgG IRdye 680 (1/7000, LI-COR, Bad Homburg, Germany). Membranes were rinsed 3 x 10 minutes in 1% PBS-Tween20 and 3 x 10 minutes in PBS before scanning on Odissey system (LI-COR, Bad Homburg, Germany). Band intensities were determined using ImageJ software as described previously.

### Results

To produce cells with an integrated target sequence, HEK-293T Cells were infected with increasing doses of LV-cPPT-PGK-mCherry-target-GFP-WPRE (MOI 1, 10, 50 and 100) and selected by FACS. Based on mCherry fluorescence, four independent populations with increasing number of integrated provirus were selected (HEK-TARGET; Figure 2A) and used for the optimization of the CRISPR system delivery. Equal amounts of LV-cPPT-CMV-hCas9-WPRE and LV-H1-sgTARGET-WPRE were incubated on each population to determine the efficiency of the CRISPR system. Cells were harvested at 3, 7, 14 and 21 days post-infection and DNA and proteins were extracted. Regions flanking the target sequence were PCR-amplified and subjected to the Surveyor mutation detection assay. In all cases, the percentage of DSB formation increased with time and is inversely proportional with the number of integrated target sequences (Figure 2B). Importantly, cell populations carrying low (12 and 32) and high (52 and 91) provirus copies can be distinguished with Surveyor results 7 days post-infection (45% and 42% compared with 37% and 35%, respectively) but the difference is reduced at 14 days post-infection (48% and 46% compared with 43%, respectively), suggesting that a plateau has been reached. For HR delivery, we used co-transfection of the CRISPR system and the AAV2-ITR-DNA-template-bGHpolyA-ITR plasmids in cell populations 1 and 10. Cells were lysed 3 and 7 days post-transfection and subjected to RFLP with the NcoI restriction site integrated after HR (Figure 1A). Data show an increased HR efficiency with time (respectively 21.7% and 18.1% for populations 1 and 10, 3 days post-transfection and 37.5% and 23.5% for populations 1 and 10 at 7 days post-transfection), which is corroborated by the expression of the fusion mCherry-GFP protein (Figure 2C-D). Finally, Applicants have evaluated the delivery of the DNA template using viral vectors in rat primary neuronal cultures. We used GFP fluorescence to assess the efficiency of gene delivery in those cells. Applicants compared AAV2/5, AAV2/6 and AAV2/DJ-ITR-CBA-GFP-WPRE-bGHpolyA-ITR. Direct GFP fluorescence reveals that AAV2/5 serotype is less efficient than AAV2/6 and AAV2/DJ in vitro (Figure 2E). Only mild differences were observed between AAV2/6 and AAV2/DJ in both HEK-293T cells and primary neurons, revealing that these two serotypes are suitable for *in vitro* HR experiments. For *in vivo* HR experiments, AAV2/5-ITR-CBA-GFP-WPRE-bGHpolyA-ITR was injected in C57B1/6 mice striatum based on previous studies (21). Direct fluorescence reveals strong GFP signal and large diffusion of the virus, confirming that this serotype is a potent gene transfer system for *in vivo* HR (Figure 2F).

### Example 3: Human HTT disruption with the CRISPR system

### Cell culture

HEK-293T cells culture, transfection and infection were performed as described in example 1 and 2.

### DNA and proteins extraction

DNA and proteins extractions using the Trizol reagent were done as described in the example 2.

### Surveyor analysis

Concentration of the extracted DNA was measured using nanodrop (Thermo Scientific, Reinach, Switzerland). Hundred nanograms of genomic DNA from HEK-293T was PCR-amplified using Kapa HiFi polymerase (KapaBiosystems, Le Mont sur Lausanne, Switzerland) with oligonucleotides located around exon 1 of the human *HTT* gene (Figure 1B, HTT-1F: TTGCTGTGTGAGGCAGAACCTGCGG, HTT-2R: TGCAGCGGCTCCTCAGCCAC). The PCR products were purified on column with the High Pure PCR product purification Kit (Roche Diagnostics GmbH, Mannheim, Germany) and eluted in 10-20 µL elution buffer. A Surveyor analysis was performed as described in example 2.

### Western blots

Western blot analysis was performed according to procedure described in the example 2.

### Mutant HTT aggregates imaging

HEK-293T Cell cultures (24 wells plates; LGC, Wesel, Germany) were grown and transfected on coverslips. Cells were fixed in 4% PFA for 10 minutes at RT, washed 2 times in PBS and mounted on microscope slides. Pictures were acquired on a Zeiss Axiovision microscope using a 10x objective and FITC channel (Carl Zeiss Microscopy GmBH, Göttingen, Germany). Constant exposure time was used to compare fluorescence of control and treated groups.

### Results

Applicants first targeted the endogenous human *HTT* gene in HEK-293T cells using the CRISPR system. We co-transfected plasmids coding for hCas9 and the sgHTT1 and assessed gene editing 3 and 7 days post-transfection. Surveyor analysis show more than 45% of DSB formation at the expected target site at 7 days post-transfection (Figure 3A). Applicants next co-transfected plasmids coding for hCas9, sgHTT1 and the fused HTT171-82Q-GFP sequence in HEK-293T cells to determine the impact of *mHTT* disruption on pathological aggregates. Western blots analysis reveal up to 90% of *mHTT* loss in treated groups when compared with control cells (Figure 3B). Epifluorescence microscopy reveals the presence of GFP-positive nuclear and cytoplasmic aggregates in non-treated cells, which disappear in cells transfected with the CRISPR system (Figure 3C). This demonstrates that *mHTT* disruption leads to the loss of pathological aggregates.

### Example 4: CRISPR system efficiency in primary neurons

### Material and methods

### Primary neuronal cultures

Rat primary neurons were cultured as described in the example 2.

For infections, 15-30ng of LV expressing GFP or HTT171-82Q-GFP or 1x10⁷ Vg of AAV2/6-CBA-GFP was incubated on neurons one day post-seeding. 12.5-20ng of LV expressing hCas9 and various sgRNA (sgGFP and sgHTT1) were co-incubated on cells 4 days post-seeding. Neurons were fixed in 4% PFA, 2-3 weeks post-seeding for fluorescence-based analysis.

### Immunohistochemistry

The following primary antibodies were used: goat polyclonal anti-GFP antibody (GFP, 1/1000 and 1/500, SicGen, Lisboa, Portugal) and mouse monoclonal anti-HTT antibody (EM48, 1/100, Millipore, Zug, Switzerland). Fixed cells were rinsed 3 x 1 minute in PBS after fixation and incubated in blocking solution (5% PBS-BSA, 0.2% Triton X100) for 20 minutes at RT. Primary antibody were incubated on cells for 1 hours at RT. Cells were washed 3 x 1 minutes in PBS and incubated in secondary antibody for 30 minutes at RT. The following secondary antibodies were used: donkey anti-goat IgG AlexaFluor 488 (1/1000, Invitrogen, Life Technologies, Zug, Switzerland) and goat anti-mouse IgG AlexaFluor 594 (1/1000, Invitrogen, Life Technologies, Zug, Switzerland). Coverslips were then washed 3 x 1 minute in PBS, incubated in DAPI for 5 minutes at RT, washed 3 x 1 minute in PBS and then mounted on a microscope slice in FluorSave (Calbiochem, Allschwill, Switzerland) medium.

### Image acquisitions

Pictures were acquired on a Zeiss Axiovision microscope using 20-40x objectives (Carl Zeiss Microscopy GmBH, Göttingen, Germany). Constant exposure time was used to compare fluorescence of control and treated groups. Number of aggregates was quantified by counting using Image J software.

### Results

The efficiency of gene editing was assessed in post-mitotic rat neurons by targeting the GFP sequence. Three days after the first infection with LV-GFP or AAV2/6-CBA-GFP, LV expressing hCas9 and Tomato-sgGFP were added to cells. Tomato fluorescence was used to visualize cells infected with the sgGFP. Two and three weeks post-infection, Tomato-positive/GFP-negative cells were observed in the culture, suggesting that GFP disruption by the CRISPR system occurred. Number of Tomato-positive/GFP-negative cells is higher at 3 weeks post-infection than at 2 weeks post-infection, suggesting that the efficiency of the system increases with time. The same experimental procedure was used to evaluate *mHTT* disruption using 15ng or 30ng of LV-Htt171-82Q-GFP at DIV1. Cells were fixed 3 weeks post-infection and stained for GFP and *mHTT* aggregates (EM48) by immunofluorescence. GFP immunohistochemistry reveals a reduced GFP signal in CRISPR treated cells than in control samples. In cells treated with the CRISPR system, number of GFP-positive aggregates is strongly reduced in both groups (15ng and 30ng of Htt171-82Q-GFP, Figure 4). Then, Applicants evaluated the combinatorial impact of multiple sgRNA targeting *mHTT.* Cells were infected with 20ng of LV-Htt171-82Q-GFP at DIV1 and then with LV-hCas9, LV-sgHTT1 and LV-sgGFP. Pictures reveal that GFP signal is less intense in cells receiving both sgHTT1 and sgGFP when compared with cells receiving only sgHTT1 3 weeks post-infection. This demonstrates that multiple sgRNA targeting the same gene could lead to an increased gene editing efficiency.

### Example 4: Delivery and efficacy of the CRISPR system in vivo

### Material and methods

### Animals

Adult males and females Tg(Drd2-EGFP)S118Gsat/Mmnc (BAC Drd2-eGFP, Mutant Mouse Regional Resource Center) between 3 and 12 months old were used for *in vivo* experiments. BAC Drd1/Drd2-eGFP double-transgenic mice were generated by crossing one male Tg(Drdla-EGFP)X60Gsat (Mutant Mouse Regional Resource Center) with one female BAC Drd2-eGFP. The animals were housed in a temperature-controlled room (22°C +- 1°C) and maintained on a 12h light/night cycle. Food and water were available *ad libitum.* All experimental procedures were performed in strict accordance with the recommendations of the European Community directive (86/609/EEC) and Swiss legislation about the care and use of laboratory animals. Sample size was chosen to take into account statistical variability due to surgical procedure based on previous studies (5).

### Stereotaxic surgery

Stereotaxic surgery was performed according to procedure described in the example 2

Concentrated viral stocks were thawed on ice and resuspended by repeated pipetting. Low doses of LV were used (100 and 200 ng p24 in PBS/1% BSA) to facilitate the analysis and avoid saturation of transcriptional activities. For co-transduction with viral vectors expressing fluorescent proteins, between 100 ng of LV-mCherry were added to the viral vector of interest. For co-infection with an AAV, 5x10⁷ Vg of AAV2/5-CBA-GFP was injected in each site.

### Brain processing

Three weeks post-lentiviral injection, the animals were killed by an overdose of sodium pentobarbital (NaCl, B-Braun, Sampach, Germany; Esconarkon, Streuli, Uznach, Germany) and transcardially perfused with a 4% PFA (Electron Microscopy Sciences, Hatfield). The brains were removed and post-fixed in 4% PFA for 12 h and then cryoprotected in 20% sucrose / PBS for 3 h and in 30% sucrose / PBS for 24 h. A sledge microtome with a freezing stage at -30 °C (Leica SM2010R, Biosystems Switzerland, Nunningen, Switzerland) was used to cut coronal sections between 20µm thickness. Slices throughout the entire striatum were collected and stored in tubes as free-floating sections in anti-freeze solution (18% sucrose, 25% sodium azide, ethylene glycol and sodium phosphate 50mM, pH = 7.4). Slices were then stored at -20°C.

### Immunohistochemistry

The following primary antibodies were used: goat polyclonal anti-mCherry antibody (mCherry, 1/1000, SicGen, Lisboa, Portugal), goat polyclonal anti-GFP antibody (GFP, 1/1000 and 1/500, SicGen, Lisboa, Portugal) and rabbit polyclonal anti-DARPP-32 antibody (DARPP-32, 1/1000, Cell Signaling, Allschwill, Switzerland). Free floating slices were rinsed 3 x 10 minutes in PBS (Laboratorium Dr Bichsel AG, Interlaken, Switzerland), followed by the saturation of non-specific sites in PBS containing 10% BSA (Sigma-Aldrich, Buchs, Switzerland) and 0.1% Triton X100 (Fluka, Sigma-Aldrich, Buchs, Switzerland) for 1h at room temperature (RT) under agitation. Primary antibodies were incubated overnight at 4°C under agitation. Slices were rinsed 3 x 10 minutes in PBS and incubated with the secondary antibodies for 1h at room temperature under agitation. The following secondary antibodies were used: donkey anti-goat IgG AlexaFluor 488 (1/1000, Invitrogen, Life Technologies, Zug, Switzerland) and goat anti-rabbit IgG AlexaFluor 594 (1/1000, Invitrogen, Life Technologies, Zug, Switzerland). Slices were then rinsed 3 x 10 minutes in PBS, followed by mounting with Vectashield mounting medium for fluorescence with DAPI (Vector Lab Inc, Burlingame). For in vitro experiments, fixed cells were rinsed 3 x 1 minute in PBS after fixation and incubated in blocking solution (5% PBS-BSA, 0.2% Triton X100) for 20 minutes at RT. Primary antibody were incubated on cells for 1 hours at RT. Cells were washed 3 x 1 minutes in PBS and incubated in secondary antibody for 30 minutes at RT. The following secondary antibodies were used: donkey anti-goat IgG AlexaFluor 488 (1/1000, Invitrogen, Life Technologies, Zug, Switzerland) and goat anti-mouse IgG AlexaFluor 594 (1/1000, Invitrogen, Life Technologies, Zug, Switzerland). Coverslips were then washed 3 x 1 minute in PBS, incubated in DAPI for 5 minutes at RT, washed 3 x 1 minute in PBS and then mounted on a microscope slice in FluorSafe (Calbiochem, Allschwill, Switzerland) medium.

### Results

Lentiviral vectors coding for hCas9 and the Tomato-sgGFP were injected separately in BAC Drd2-eGFP transgenic mice expressing eGFP in D2 neurons of the striatum to evaluate toxicity of the system. GFP expression was used as an indirect marker of neuronal toxicity. LV expressing mCherry reporter gene under the PGK promoter was co-injected to visualize the infected area (half amount of hCas9 or Tomato-sgGFP). Based on both mCherry and GFP fluorescence, no toxicity of the constructs was observed. To evaluate the efficiency of the CRISPR system in vivo, BAC Drd1-eGFP and BAC Drd2-eGFP were crossed to generate BAC Drd1/Drd2-eGFP double-transgenic mice expressing eGFP in medium-sized spiny neurons of the direct and indirect pathway. Equal amount of LV expressing hCas9 and Tomato-sgGFP were co-injected into the striatum of mice. Brain were fixed and analyzed for direct fluorescence 3 weeks post-infection. Pictures reveal that Tomato-positive cells are GFP-negative, whereas cells out of the infected area are still GFP. DARPP-32 staining reveals that GFP-negative neurons are still functional, confirming that loss of GFP signal is due to gene disruption. Additionally, co-infection of AAV2/5-CBA-GFP, LV-hCas9 and LV-Tomato-sgGFP results in the loss of GFP in the injected site of LV, with Tomato-positive/GFP-negative cells. This reveals that the CRISPR system is efficient to disrupt an exogenous DNA sequence *in vivo.*

### REFERENCES

1. Vonsattel JP & DiFiglia M (1998) Huntington disease. J Neuropath Exp Neurol 57:369-384.
2. Zuccato C, Valenza M, & Cattaneo E (2010) Molecular mechanisms and potential therapeutical targets in Huntington's disease. Physiol Rev 90(3):905-981.
3. Ross CA & Tabrizi SJ (2011) Huntington's disease: from molecular pathogenesis to clinical treatment. Lancet Neurol 10(1):83-98.
4. Fiszer A & Krzyzosiak WJ (2014) Oligonucleotide-based strategies to combat polyglutamine diseases. Nucleic Acids Res.
5. Drouet V, et al. (2009) Sustained effects of nonallele-specific Huntingtin silencing. Ann Neurol 65(3):276-285.
6. Garriga-Canut M, et al. (2012) Synthetic zinc finger repressors reduce mutant huntingtin expression in the brain of R6/2 mice. Proc Natl Acad Sci U S A 109(45):E3136-3145.
7. Jinek M, et al. (2013) RNA-programmed genome editing in human cells. eLife 2:e00471.
8. Sternberg SH, Redding S, Jinek M, Greene EC, & Doudna JA (2014) DNA interrogation by the CRISPR RNA-guided endonuclease Cas9. Nature*.*
9. Merienne N, Le Douce J, Faivre E, Deglon N, & Bonvento G (2013) Efficient gene delivery and selective transduction of astrocytes in the mammalian brain using viral vectors. Front Cell Neurosci 7:106.
10. Simonato M, et al. (2013) Progress in gene therapy for neurological disorders. Nat Rev Neurol 9(5):277-291.
11. Cartier N, et al. (2009) Hematopoietic stem cell gene therapy with a lentiviral vector in X-linked adrenoleukodystrophy. Science 326(5954):818-823.
12. Palfi S, et al. (2014) Long-term safety and tolerability of ProSavin, a lentiviral vector-based gene therapy for Parkinson's disease: a dose escalation, open-label, phase 1/2 trial. Lancet 383(9923):1138-1146.
13. Weinberg MS, Samulski RJ, & McCown TJ (2013) Adeno-associated virus (AAV) gene therapy for neurological disease. Neuropharmacology 69:82-88.
14. Naldini L, et al. (1996) In vivo gene delivery and stable transduction of nondividing cells by a lentiviral vector. Science 272(5259):263-267.
15. Mali P, et al. (2013) RNA-guided human genome engineering via Cas9. Science 339(6121):823-826.
16. Deglon N, et al. (2000) Self-inactivating lentiviral vectors with enhanced transgene expression as potential gene transfer system in Parkinson's disease. Hum Gene Ther 11(1):179-190.
17. Hottinger AF, Azzouz M, Deglon N, Aebischer P, & Zurn AD (2000) Complete and long-term rescue of lesioned adult motoneurons by lentiviral-mediated expression of glial cell line-derived neurotrophic factor in the facial nucleus. J Neurosci 20(15):5587-5593.
18. Pereira de Almeida L, Ross CA, Zala D, Aebischer P, & Deglon N (2002) Lentiviral-mediated delivery of mutant huntingtin in the striatum of rats induces a selective neuropathology modulated by polyglutamine repeat size, huntingtin expression levels, and protein length. Journal of Neuroscience 22(9):3473-3483.
19. Dull T, et al. (1998) A third-generation lentivirus vector with a conditional packaging system. J Virol 72(11):8463-8471.
20. Low K, Aebischer P, & Schneider BL (2013) Direct and retrograde transduction of nigral neurons with AAV6, 8, and 9 and intraneuronal persistence of viral particles. Hum Gene Ther 24(6):613-629.
21. Drinkut A, Tereshchenko Y, Schulz JB, Bahr M, & Kugler S (2012) Efficient gene therapy for Parkinson's disease using astrocytes as hosts for localized neurotrophic factor delivery. Mol Ther 20(3):534-543.
22. Melo SP, et al. (2014) Somatic Correction of Junctional Epidermolysis Bullosa by a Highly Recombinogenic AAV Variant. Mol Ther 22(4):725-733.
23. Aurnhammer C, et al. (2012) Universal real-time PCR for the detection and quantification of adeno-associated virus serotype 2-derived inverted terminal repeat sequences. Hum Gene Ther Methods 23(1):18-28.
24. Qiu P, et al. (2004) Mutation detection using Surveyor nuclease. Biotechniques 36(4):702-707.
25. Ran FA, et al. (2013) Genome engineering using the CRISPR-Cas9 system. Nat Protoc 8(11):2281-2308.
26. Drouet V, et al. (2014) Allele-specific silencing of mutant huntingtin in rodent and human stem cells. PlosOne (In Revision).

## Claims

1. A kit for the treatment of Huntington's disease (HD) for allele or non-allele-specific huntingtin (*HTT*) gene editing or repair comprising:
a gene delivery vector consisting of at least one viral vector selected among adeno-associated vectors (AAV) and/or lentiviral vectors (LV);
a Cas9 being human codon-optimized ;
an artificial single guide RNA (sgRNA) having a total size from 63-115 nucleotides comprising a crRNA sequence of 15-30 nucleotides recognizing the *HTT* gene and capable of targeting around the ATG +/- 4 kb of the *HTT* gene wherein said sgRNA further comprises a tracr sequence of 48-85 nucleotides long.

2. The kit for the treatment of Huntington's disease of claim 1, **characterized in that** said gene delivery vector contains various expression cassettes comprising promoters and/or miRNA-regulated system so as to modulate transgene expression.

3. The kit for the treatment of Huntington's disease according to any of claims 1-2, **characterized in that** said AAV is a mutant vector.

4. The kit for the treatment of Huntington's disease according claim 3, **characterized in that** said AAV is the AAV-DJ.

5. The kit for the treatment of Huntington's disease according to any of claims 1-4, **characterized in that** said Cas9 is mutated to improve the efficiency and safety.

6. The kit for the treatment of Huntington's disease according claim 5, **characterized in that** said mutated Cas9 is a Cas9 nickase.

7. The kit for the treatment of Huntington's disease according to any of claims 1-6, **characterized in that** gene delivery vector consist in a single vector or separated vectors.

8. The kit for the treatment of Huntington's disease according to any of claims 1-7, **characterized in that** said kit comprises multiple transcriptionally independent sgRNA targeting the *HTT* gene.

9. The kit for the treatment of Huntington's disease according to any of claims 1-9, **characterized in that** said artificial sgRNA comprising a crRNA sequence of 15-30 nucleotides recognizing the *HTT* gene is human-specific or a conserved sequence between human, rodent and primates with a maximum tolerated number of mismatches between sgRNA and *HTT* gene of 3 in the first 8 nucleotides of the sgRNA.

10. The kit for the treatment of Huntington's disease according to any of claims 1-9, **characterized in that** said artificial crRNA sequence of 15-30 nucleotides is encoded by the sequences selected among the group of SEQ ID NO: 2-7.

11. The kit for the treatment of Huntington's disease according to any of claims 1-10, further comprising an exogenous DNA template for homologous recombination (HR) of human *HTT.*

12. The kit for the treatment of Huntington's disease according to any of claims 1-10, for use in a method for non-allele-specific *HTT* inactivation of the human *HTT* wild-type and mutant alleles.

13. The kit for the treatment of Huntington's disease according to any of claims 1-11, for use in a method for non-allele-specific *HTT* gene repair based on HR with a DNA template containing a wild-type *HTT* sequence.

14. The kit for the treatment of Huntington's disease according to any of claims 1-10, for use in a method for allele-specific *HTT* inactivation of the human mutant *HTT* gene **characterized in that** the sgRNA of said kit is capable of recognizing SNP sequences located around the ATG of the *HTT* gene of +/- 4 kb and with a high frequency of heterozygosity in the human population.

15. The kit for the treatment of Huntington's disease according to any of claims 1-11, for use in a method for mutant *HTT* gene repair based on HR with a DNA template containing a wild-type *HTT* sequence **characterized in that** the sgRNA of said kit is capable of recognizing SNP sequences located around the ATG of the *HTT* gene of +/- 4 kb and with a high frequency of heterozygosity in the human population.
